# EUROPEAN PATENT SPECIFICATION

(11) **EP 3 146 075 B1**
(45) Date of publication and mention of the grant of the patent: **17.07.2019**
(21) Application number: 15795415.7
(22) Date of filing: 18.05.2015
(51) Int. Cl.: C12Q 1/6869

(54) **ION SENSOR DNA AND RNA SEQUENCING BY SYNTHESIS USING NUCLEOTIDE REVERSIBLE TERMINATORS**
IONENSENSOR-DNA- UND -RNA-SEQUENZIERUNG DURCH SYNTHESE UNTER VERWENDUNG NUKLEOTIDREVERSIBLER TERMINATOREN
SÉQUENÇAGE D'ADN ET D'ARN PAR SYNTHÈSE BASÉ SUR LA DÉTECTION D'IONS À L'AIDE DE TERMINATEURS NUCLÉOTIDIQUES RÉVERSIBLES

(30) Priority: 19.05.2014 US 201462000306 P
(43) Date of publication of application: 29.03.2017
(73) Proprietor: The Trustees of Columbia University in the City of New York, New York, NY 10027 (US)
(72) Inventor: JU, Jingyue, Englewood Cliffs, NJ 07632 (US); RUSSO, James J., New York, NY 10032 (US); YU, Lin, Flushing, NY 11355 (US)
(74) Representative: Almond-Martin, Carol
(86) International application number: PCT/US2015/031358
(87) International publication number: WO 2015/179284

(56) References cited:
- US-A1- 2010 137 143
- US-A1- 2012 052 489
- US-A1- 2012 052 489
- US-A1- 2013 264 207
- US-B1- 7 270 951
- J. GUO ET AL: "Four-color DNA sequencing with 3'-O-modified nucleotide reversible terminators and chemically cleavable fluorescent dideoxynucleotides", PROCEEDINGS OF THE NATIONAL ACADEMY OF SCIENCES, vol. 105, no. 27, 1 January 2008 (2008-01-01), pages 9145-9150, XP55013741, ISSN: 0027-8424, DOI: 10.1073/pnas.0804023105

### SEQUENCE LISTING

<110> The Trustees of Columbia University in the City of New York Ju, Jingyue Russo, James J. Yu, Lin
<120> ION SENSOR DNA AND RNA SEQUENCING BY SYNTHESIS USING NUCLEOTIDE REVERSIBLE TERMINATORS
<130> 0575/82337-PCT/JPW/GJG/JAK
<150> US 62/000,306
   <151> 2014-05-19
<160> 1
<170> PatentIn version 3.5
<210> 1
   <211> 69
   <212> DNA
   <213> Artificial
<220>
   <223> synthesized oligonucleotide
<400> 1
   tttttttttt aggaaccctt ggccaaattt ttcccccgga aacagctatg accggtcata        60
   gctgtttcc                                                                69

## Description

Throughout this application, certain publications are referenced in parentheses. Full citations for these publications may be found immediately preceding the claims.

### Background of the Invention

High-throughput sequencing has become a basic support technology for essentially all areas of modern biology, from arenas as disparate as ecology and evolution to gene discovery and personalized medicine. Through the use of massively parallel sequencing in all its varieties, it is possible to identify homology among genes throughout the tree of life, to detect single nucleotide polymorphisms (SNPs), copy number variants, and genomic rearrangements in individual humans; to characterize in detail the transcriptome and its transcription factor binding sites; and to provide a detailed and even global view of the epigenome (Hawkins et al. 2010; Morozova et al. 2009; Park et al. 2009).

In order to move the field of personalized medicine forward, it will be essential to garner complete genotype and phenotype information for representative samples of all geo-ethnic population groups, including individuals presenting with a broad range of complex diseases. Having such a compendium of data will eventually permit physicians to tailor treatment to each patient, taking into account genetic factors controlling their ability to tolerate and respond to different pharmaceuticals. This will require, however, the cost of whole genome sequencing to be in the range of most other medical tests, generally taken to be $1,000 or less, and to have a lower error rate per base than the frequency of all but the rarest SNPs (<1 in 10,000) (Fuller et al. 2009; Ng et al. 2010; Shen et al. 2010).

A variety of recent so-called "next generation" sequencing technologies have brought down the cost of sequencing a genome with relatively high accuracy close to $100,000, but this is still prohibitive for health care systems even in the most affluent countries. Further efficiencies in current technologies and the introduction of breakout technologies are required to move the field to the $1,000 goal. Among the "next generation" sequencing technologies, the most popular has been the sequencing by synthesis (SBS) strategy (Fuller et al. 2009) which underlies such diverse instruments as those commercialized or in development by companies such as Roche, Illumina, Helicos, and Intelligent BioSystems. One successful SBS approach involves the use of fluorescently labeled nucleotide reversible terminators (NRTs) (Ju et al. 2003; Li et al. 2003; Ruparel et al. 2005; Seo et al. 2005; Ju et al. 2006). These are modified dNTPs (A, C, T/U and G) that have both a base-specific fluorophore and a moiety blocking the 3' hydroxyl group of the sugar and thereby impeding its extension by the next nucleotide attached to each dNTP via a chemically, enzymatically, or photo-cleavable bond. This permits one to interrupt the polymerase reaction, determine the base incorporated according to the color of the attached fluorescent tag, and then remove both the fluor and the 3'-OH blocking group, to permit one more base to be added. The importance of the use of NRTs is that they greatly reduce the possibility of read-ahead due to the addition of more than one nucleotide, especially with the use of intermediate synchronization strategies. Both Roche's pyrosequencing approach (Ronaghi et al. 1998) and Helicos' use of "virtual" terminators (Bowers et al. 2009; Harris et al. 2008) require the addition of each base, one by one, followed by a readout that is indirect (light production in the former), or direct but single color (in the latter) . Despite the undeniable power of these methods (long read length for Roche, single molecule capability for Helicos), the methods have difficulty in accurately decoding homopolymer stretches longer than ∼4 or 5 bases (Ronaghi et al. 2001). Further, pyrosequencing suffers from false positives, as free dNTPs will spontaneously decompose in solution, releasing a pyrophosphate (Gerstein 2001), producing a signal.

Recently, Ion Torrent, Inc., has described sequencing strategies in which the proton released as each nucleotide is incorporated into the DNA chain is captured by an ion sensor and digitized using semiconductor technology (Anderson et al. 2009; Rothberg et al. 2011). Again, however, since this output is identical no matter which of the four nucleotides is incorporated, because these strategies use natural nucleotides, this necessitates the base-by-base addition strategy, with its inherent difficulty in achieving accurate reads through homopolymeric base runs.

An SBS method has been described in which each nucleotide has a unique Raman spectroscopy peak, wherein determination of the wavenumber of the Raman peak is used to identify an incorporated nucleotide analogue (PCT International Application Publication No. WO 2012/162429). However, using Raman spectroscopy to detect and identify nucleotide analogues suffers from low sensitivity inherent in this technique.

Rothberg et al. (U.S. Patent Application Publication No. 2010/137143 A1, published on 3 June 2010) discloses methods and apparatus relating to FET arrays including large FET arrays for monitoring chemical and/or biological reactions such as nucleic acid sequencing-by-synthesis reactions. Rothberg et al. does not disclose a sequencing method using hydrogen ion detection and nucleotides with blocking groups that are a hydrocarbyl, or substituted hydrocarbyl having a mass of less than 300 daltons and which is photochemically cleavable.

Gordon Steven; Olejnik Jerzey (U.S. Patent Application Publication No. 2012/0052489 A1, published on March 1, 2012) discloses methods and compositions, and systems for determining the identity of nucleic acids in nucleotide sequences, including sequences with one or more homopolymer ranges. Gordon Steven; Olejnik Jerzey does not disclose a sequencing method using hydrogen ion detection and nucleotides with blocking groups that are a hydrocarbyl, or substituted hydrocarbyl having a mass of less than 300 daltons and which is photochemically cleavable.

### Summary of the Invention

This invention is directed to a method for determining the sequence of consecutive nucleotide residues in a single-stranded DNA in a solution comprising:
(a) contacting the single-stranded DNA, having a primer hybridized to a portion thereof, with a DNA polymerase and a deoxyribonucleotide triphosphate (dNTP) analogue base under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 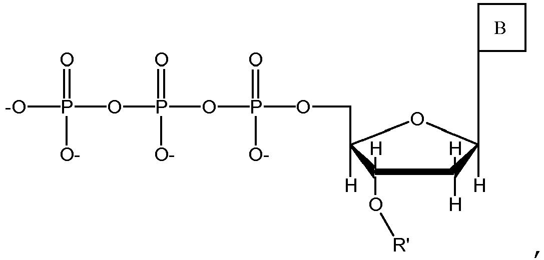 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the primer to form a DNA extension product, and if so, determining from the identity of the incorporated dNTP analogue the identity of the nucleotide residue in the single-stranded DNA complementary thereto, thereby determining the identity of the nucleotide residue in the single-stranded DNA, and wherein no change in hydrogen ion concentration indicates that the dNTP analogue has not been incorporated into the primer in step (a) ;
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded DNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded DNA which is immediately 5' to a nucleotide residue of the single-stranded DNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded DNA so as to thereby determine the sequence of the consecutive nucleotide residues of the DNA.

This invention is directed to a method for determining the sequence of consecutive nucleotide residues in a single-stranded RNA in a solution comprising:
(a) contacting the single-stranded RNA, having an RNA primer hybridized to a portion thereof, with a RNA polymerase and a ribonucleotide triphosphate (rNTP) analogue under conditions permitting the RNA polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the RNA primer, so as to form an RNA extension product, wherein (1) the rNTP analogue has the structure: 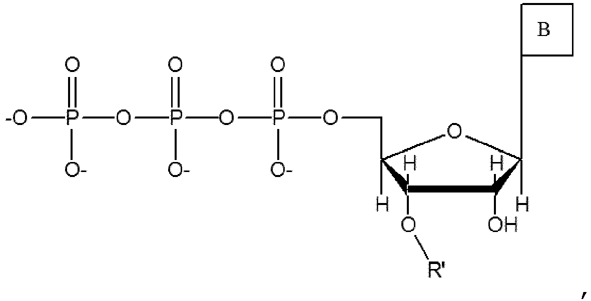 wherein B is a base and is adenine, guanine, cytosine, or uracil, and (2) R' is a hydrocarbyl, or a substituted hydrocarbyl, having a mass of less than 300 daltons and which is photochemically cleavable;
(b) determining whether incorporation of the rNTP analogue has occurred in step (a) by detecting an increase in hydrogen ion concentration of the solution, wherein an increase in hydrogen ion concentration indicates that the rNTP analogue has been incorporated into the RNA primer to form an RNA extension product, and if so, determining from the identity of the incorporated rNTP analogue the identity of the nucleotide residue in the single-stranded RNA complementary thereto, thereby determining the identity of the nucleotide residue in the single-stranded RNA, and wherein no change in hydrogen ion concentration indicates that the rNTP analogue has not been incorporated into the RNA primer in step (a);
(c) if no change in hydrogen ion concentration has been detected in step (b), iteratively performing steps (a) and (b), wherein in each iteration of step (a) for a given nucleotide residue, the identity of which is being determined, the rNTP analogue comprises a base which is a different type of base from the type of base of the rNTP analogues in every preceding iteration of step (a) for that nucleotide residue, until an rNTP analogue is incorporated into the RNA primer to form an RNA extension product, and determining from the identity of the incorporated rNTP analogue the identity of the nucleotide residue in the single-stranded RNA complementary thereto, thereby determining the identity of the nucleotide residue in the single-stranded RNA;
(d) if an increase in hydrogen ion concentration has been detected and an rNTP analogue is incorporated, subsequently treating the incorporated rNTP analogue with light to photochemically cleave the R' group so as to replace the R' group thereof with an H atom thereby providing a 3' OH group at the 3' terminal of the RNA extension product; and
(e) iteratively performing steps (a) to (d), as necessary, for each nucleotide residue of the consecutive nucleotide residues of the single-stranded RNA to be sequenced, except that in each repeat of step (a) the rNTP analogue is (i) incorporated into the RNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the RNA extension product resulting from a preceding iteration of step (a) or step (c), so as to form a subsequent RNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

This invention is directed to a method for determining the sequence of consecutive nucleotide residues in a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 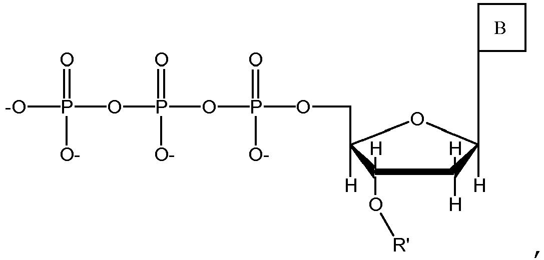 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the DNA primer to form a DNA extension product, and if so, determining from the identity of the incorporated dNTP analogue the identity of the nucleotide residue in the single-stranded RNA complementary thereto, thereby determining the identity of the nucleotide residue in the single-stranded RNA, and wherein no change in hydrogen ion concentration indicates that the dNTP analogue has not been incorporated into the DNA primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded RNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

The specification discloses a method for determining the identity of a nucleotide residue of a single-stranded DNA in a solution comprising:
(a) contacting the single-stranded DNA, having a primer hybridized to a portion thereof, with a DNA polymerase and a deoxyribonucleotide triphosphate (dNTP) analogue under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 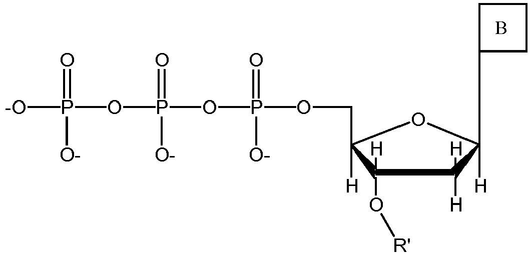 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the dNTP analogue into the primer to form a DNA extension product has occurred in step (a) by determining if an increase in hydrogen ion concentration of the solution has occured, wherein
   (i) if the dNTP analogue has been incorporated into the primer, determining from the identity of the incorporated dNTP analogue the identity of the nucleotide residue in the single-stranded DNA complementary thereto, thereby determining the identity of the nucleotide residue in the single-stranded DNA, and
   (ii) if no change in hydrogen ion concentration has occurred, iteratively performing step (a), wherein in each iteration of step (a) the dNTP analogue comprises a base which is a different type of base from the type of base of the dNTP analogues in every preceding iteration of step (a), until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA.

The specification discloses a method for determining the sequence of consecutive nucleotide residues in a single-stranded DNA in a solution comprising:
(a) contacting the single-stranded DNA, having a primer hybridized to a portion thereof, with a DNA polymerase and a deoxyribonucleotide triphosphate (dNTP) analogue under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 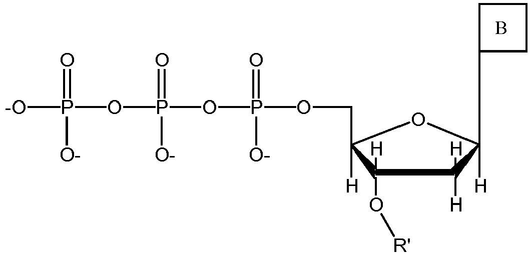 wherein B is a base and is adenine, guanine, cytosine, or thymine, and (2) R' is (i) or -CH₂N₃, or 2-nitrobenzyl, or (ii) is a hydrocarbyl, or a substituted hydrocarbyl, having a mass of less than 300 daltons;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the primer to form a DNA extension product, and if so, determining from the identity of the incorporated dNTP analogue the identity of the nucleotide residue in the single-stranded DNA complementary thereto, thereby determining the identity of the nucleotide residue in the single-stranded DNA, and wherein no change in hydrogen ion concentration indicates that the dNTP analogue has not been incorporated into the primer in step (a) ;
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA;
(d) if an increase in hydrogen ion concentration has been detected and a dNTP analogue is incorporated, subsequently treating the incorporated dNTP nucleotide analogue so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded DNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded DNA which is immediately 5' to a nucleotide residue of the single-stranded DNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded DNA so as to thereby determine the sequence of the consecutive nucleotide residues of the DNA.

The specification discloses a method for determining the identity of a nucleotide residue of a single-stranded RNA in a solution comprising:
(a) contacting the single-stranded RNA, having an RNA primer hybridized to a portion thereof, with a polymerase and a ribonucleotide triphosphate (rNTP) analogue under conditions permitting the polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the primer, so as to form an RNA extension product, wherein (1) the rNTP analogue has the structure: 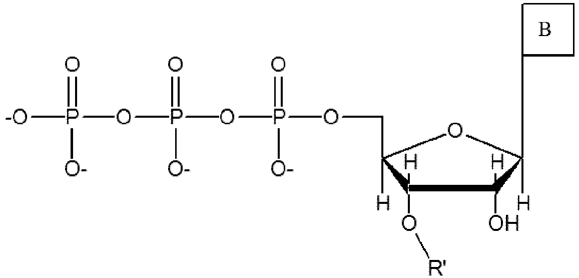 wherein B is a base and is adenine, guanine, cytosine, or uracil, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the rNTP analogue into the RNA primer to form an RNA extension product has occurred in step (a) by determining if an increase in hydrogen ion concentration of the solution has occured, wherein
   (i) if the rNTP analogue has been incorporated into the RNA primer, determining from the identity of the incorporated rNTP analogue the identity of the nucleotide residue in the single-stranded RNA complementary thereto, thereby determining the identity of the nucleotide residue in the single-stranded RNA, and
   (ii) if no change in hydrogen ion concentration has occurred, iteratively performing step (a), wherein in each iteration of step (a) the rNTP analogue comprises a base which is a different type of base from the type of base of the rNTP analogues in every preceding iteration of step (a), until an rNTP analogue is incorporated into the RNA primer to form an RNA extension product, and determining from the identity of the incorporated rNTP analogue the identity of the nucleotide residue in the single-stranded RNA complementary thereto, thereby determining the identity of the nucleotide residue in the single-stranded RNA.

The specification discloses a method for determining the sequence of consecutive nucleotide residues in a single-stranded RNA in a solution comprising:
(a) contacting the single-stranded RNA, having an RNA primer hybridized to a portion thereof, with a polymerase and a ribonucleotide triphosphate (rNTP) analogue under conditions permitting the polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the RNA primer, so as to form an RNA extension product, wherein (1) the rNTP analogue has the structure: 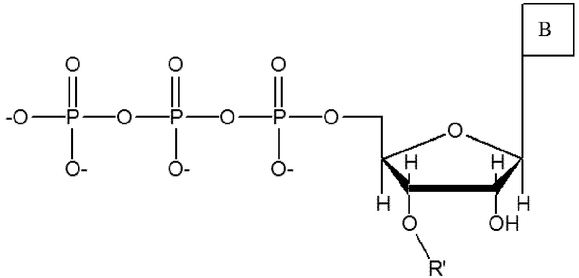 wherein B is a base and is adenine, guanine, cytosine, or uracil, and (2) R' is (i)-CH₂N₃, or 2-nitrobenzyl, or (ii) is a hydrocarbyl, or a substituted hydrocarbyl, having a mass of less than 300 daltons;
(b) determining whether incorporation of the rNTP analogue has occurred in step (a) by detecting an increase in hydrogen ion concentration of the solution, wherein an increase in hydrogen ion concentration indicates that the rNTP analogue has been incorporated into the RNA primer to form an RNA extension product, and if so, determining from the identity of the incorporated rNTP analogue the identity of the nucleotide residue in the single-stranded RNA complementary thereto, thereby determining the identity of the nucleotide residue in the single-stranded RNA, and wherein no change in hydrogen ion concentration indicates that the rNTP analogue has not been incorporated into the RNA primer in step (a);
(c) if no change in hydrogen ion concentration has been detected in step (b), iteratively performing steps (a) and (b), wherein in each iteration of step (a) for a given nucleotide residue, the identity of which is being determined, the rNTP analogue comprises a base which is a different type of base from the type of base of the rNTP analogues in every preceding iteration of step (a) for that nucleotide residue, until an rNTP analogue is incorporated into the primer to form an RNA extension product, and determining from the identity of the incorporated rNTP analogue the identity of the nucleotide residue in the single-stranded RNA complementary thereto, thereby determining the identity of the nucleotide residue in the single-stranded RNA;
(d) if an increase in hydrogen ion concentration has been detected and an rNTP analogue is incorporated, subsequently treating the incorporated rNTP nucleotide analogue so as to replace the R' group thereof with an H atom thereby providing a 3' OH group at the 3' terminal of the RNA extension product; and
(e) iteratively performing steps (a) to (d), as necessary, for each nucleotide residue of the consecutive nucleotide residues of the single-stranded RNA to be sequenced, except that in each repeat of step (a) the rNTP analogue is (i) incorporated into the RNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the RNA extension product resulting from a preceding iteration of step (a) or step (c), so as to form a subsequent RNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

The specification discloses a method for determining the identity of a nucleotide residue of a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the DNA primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 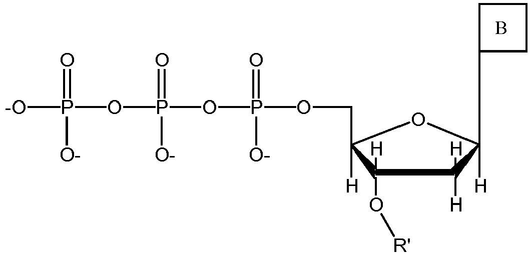 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the dNTP analogue into the DNA primer to form a DNA extension product has occurred in step (a) by determining if an increase in hydrogen ion concentration of the solution has occured, wherein
   (i) if the dNTP analogue has been incorporated into the DNA primer, determining from the identity of the incorporated dNTP analogue the identity of the nucleotide residue in the single-stranded RNA complementary thereto, thereby determining the identity of the nucleotide residue in the single-stranded RNA, and
   (ii) if no change in hydrogen ion concentration has occurred, iteratively performing step (a), wherein in each iteration of step (a) the dNTP analogue comprises a base which is a different type of base from the type of base of the dNTP analogues in every preceding iteration of step (a), until a dNTP analogue is incorporated into the DNA primer to form a DNA extension product, and determining from the identity of the incorporated dNTP analogue the identity of the nucleotide residue in the single-stranded DNA complementary thereto, thereby determining the identity of the nucleotide residue in the single-stranded DNA.

The specification discloses a method for determining the sequence of consecutive nucleotide residues in a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 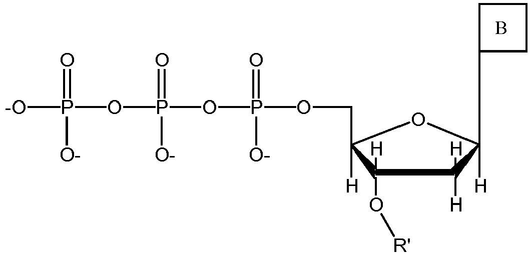 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the DNA primer to form an RNA extension product, and if so, determining from the identity of the incorporated dNTP analogue the identity of the nucleotide residue in the single-stranded RNA complementary thereto, thereby determining the identity of the nucleotide residue in the single-stranded RNA, and wherein no change in hydrogen ion concentration indicates that the dNTP analogue has not been incorporated into the DNA primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA;
(d) if an increase in hydrogen ion concentration has been detected and a dNTP analogue is incorporated, subsequently treating the incorporated dNTP nucleotide analogue so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded RNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

### BRIEF DESCRIPTION OF THE FIGURES

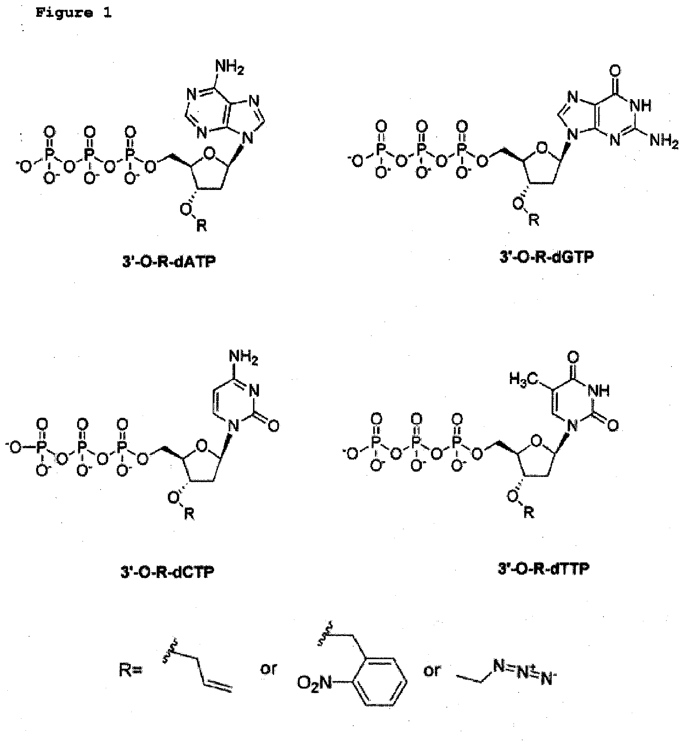

**Fig. 1****.** NRTs with various blocking groups (R) at the 3'-OH position. Photo-cleavage of 2-nitrobenzyl group (lower center) or chemical cleavage of allyl (lower left) and azidomethyl groups (lower right) restores the 3'-OH for subsequent reaction cycles.

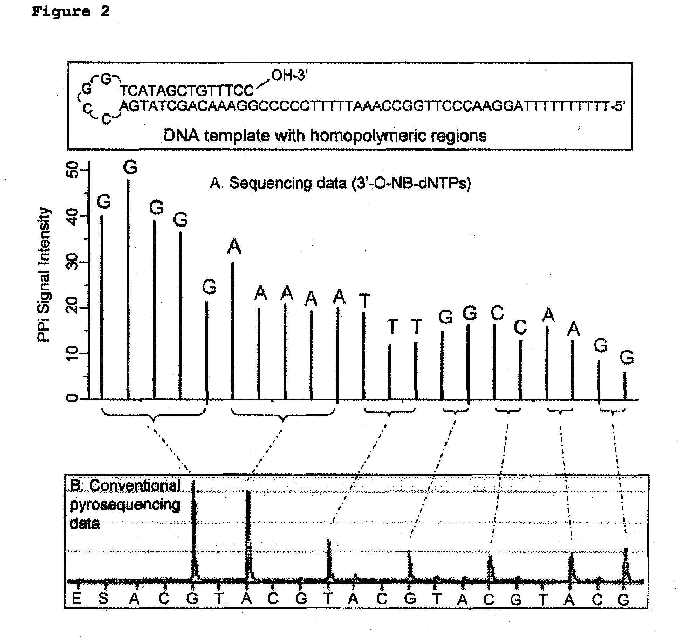

**Fig. 2****.** Comparison of reversible terminator-pyrosequencing of DNA using 3'-O-(2-nitrobenzyl)-dNTPs with conventional pyrosequencing using natural nucleotides (NB = 2-nitrobenzyl). (A) The self-priming DNA template with stretches of homopolymeric regions (5 C's, 5 T's, 3 A's, 2 C's, 2 G's, 2 T's and 2 C's) was sequenced using 3'-O-(2-nitrobenzyl)-dNTPs. The homopolymeric regions are clearly identified with each peak corresponding to the identity of each base in the DNA template. (B) Pyrosequencing data using natural nucleotides. The homopolymeric regions produced two large peaks corresponding to the stretches of G's and A's and 5 smaller peaks corresponding to stretches of T's, G's, C's, A's and G's. However, it is very difficult to decipher the exact sequence from the data.

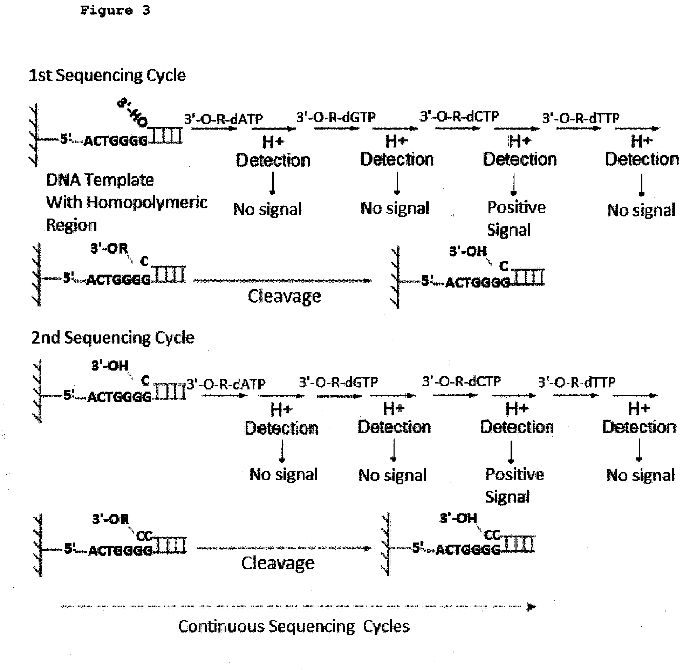

**Fig. 3****.** Ion Sensor Sequencing By Synthesis (SBS) with NRTs. Surface-attached templates are extended with NRTs, added one at a time. If there is incorporation, a H+ ion is released and detected. After cleavage of the blocking group, the next cycle is initiated. Because the NRTs force the reactions to pause after each cycle, the lengths of homopolymers are determined with precision.

### Detailed Description of the Invention

The present invention is directed to a method for determining the sequence of consecutive nucleotide residues in a single-stranded DNA in a solution comprising:
(a) contacting the single-stranded DNA, having a primer hybridized to a portion thereof, with a DNA polymerase and a deoxyribonucleotide triphosphate (dNTP) analogue base under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 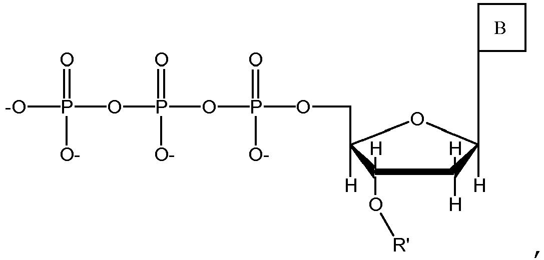 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the primer to form a DNA extension product, and if so, determining from the identity of the incorporated dNTP analogue the identity of the nucleotide residue in the single-stranded DNA complementary thereto, thereby determining the identity of the nucleotide residue in the single-stranded DNA, and wherein no change in hydrogen ion concentration indicates that the dNTP analogue has not been incorporated into the primer in step (a) ;
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded DNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded DNA which is immediately 5' to a nucleotide residue of the single-stranded DNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded DNA so as to thereby determine the sequence of the consecutive nucleotide residues of the DNA.

The present invention is also directed to a method for determining the sequence of consecutive nucleotide residues in a single-stranded RNA in a solution comprising:
(a) contacting the single-stranded RNA, having an RNA primer hybridized to a portion thereof, with a RNA polymerase and a ribonucleotide triphosphate (rNTP) analogue under conditions permitting the RNA polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the RNA primer, so as to form an RNA extension product, wherein (1) the rNTP analogue has the structure: 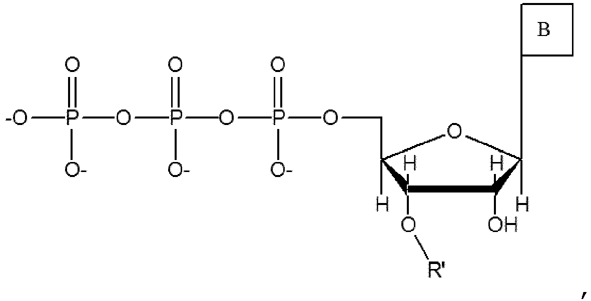 wherein B is a base and is adenine, guanine, cytosine, or uracil, and (2) R' is a hydrocarbyl, or a substituted hydrocarbyl, having a mass of less than 300 daltons and which is photochemically cleavable;
(b) determining whether incorporation of the rNTP analogue has occurred in step (a) by detecting an increase in hydrogen ion concentration of the solution, wherein an increase in hydrogen ion concentration indicates that the rNTP analogue has been incorporated into the RNA primer to form an RNA extension product, and if so, determining from the identity of the incorporated rNTP analogue the identity of the nucleotide residue in the single-stranded RNA complementary thereto, thereby determining the identity of the nucleotide residue in the single-stranded RNA, and wherein no change in hydrogen ion concentration indicates that the rNTP analogue has not been incorporated into the RNA primer in step (a);
(c) if no change in hydrogen ion concentration has been detected in step (b), iteratively performing steps (a) and (b), wherein in each iteration of step (a) for a given nucleotide residue, the identity of which is being determined, the rNTP analogue comprises a base which is a different type of base from the type of base of the rNTP analogues in every preceding iteration of step (a) for that nucleotide residue, until an rNTP analogue is incorporated into the RNA primer to form an RNA extension product, and determining from the identity of the incorporated rNTP analogue the identity of the nucleotide residue in the single-stranded RNA complementary thereto, thereby determining the identity of the nucleotide residue in the single-stranded RNA;
(d) if an increase in hydrogen ion concentration has been detected and an rNTP analogue is incorporated, subsequently treating the incorporated rNTP analogue with light to photochemically cleave the R' group so as to replace the R' group thereof with an H atom thereby providing a 3' OH group at the 3' terminal of the RNA extension product; and
(e) iteratively performing steps (a) to (d), as necessary, for each nucleotide residue of the consecutive nucleotide residues of the single-stranded RNA to be sequenced, except that in each repeat of step (a) the rNTP analogue is (i) incorporated into the RNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the RNA extension product resulting from a preceding iteration of step (a) or step (c), so as to form a subsequent RNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

The present invention is also directed to a method for determining the sequence of consecutive nucleotide residues in a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 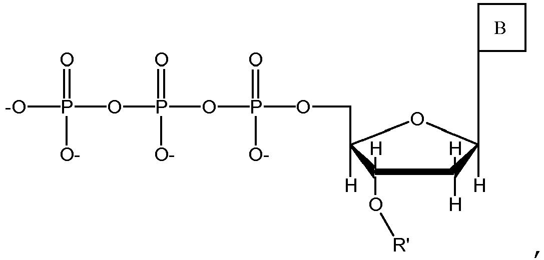 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the DNA primer to form a DNA extension product, and if so, determining from the identity of the incorporated dNTP analogue the identity of the nucleotide residue in the single-stranded RNA complementary thereto, thereby determining the identity of the nucleotide residue in the single-stranded RNA, and wherein no change in hydrogen ion concentration indicates that the dNTP analogue has not been incorporated into the DNA primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded RNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

In a further embodiment of the methods of the invention, wherein UV light is used to treat the R' group of a dNTP analogue or rNTP analogue incorporated into a primer or DNA or RNA extension product so as to photochemically cleave the moiety attached to the 3'-O so as to replace the 3'-OR' with a 3'-OH.

In a further embodiment of the methods of the invention, R' is a substituted hydrocarbyl, and is a nitrobenzyl.

In a further embodiment of the methods of the invention, R' is a 2-nitrobenzyl, and wherein UV light is used to treat the R' group of a dNTP analogue or rNTP analogue incorporated into a primer or DNA or RNA extension product so as to photochemically cleave the moiety attached to the 3'-O so as to replace the 3'-O-R' with a 3'-OH, and preferably the light is ∼355nm irradiation.

In a further embodiment of the methods of the invention, the single-stranded DNA or RNA is in a solution in a reaction chamber disposed on a sensor which is (i) formed in a semiconductor substrate and (ii) comprises a field-effect transistor or chemical field-effect transistor configured to provide at least one output signal in response to an increase in hydrogen ion concentration of the solution resulting from the formation of a phosphodiester bond between a dNTP or dNTP analogue or rNTP or rNTP analogue and a primer or a DNA or RNA extension product.

In a further embodiment of the methods of the invention, the reaction chamber is one of a plurality of reaction chambers disposed on a sensor array formed in a semiconductor substrate and comprised of a plurality of sensors, each reaction chamber being disposed on at least one sensor and each sensor of the array comprising a field-effect transistor, or a chemical field-effect transistor, configured to provide at least one output signal in response to an increase in hydrogen ion concentration of the solution resulting from the formation of a phosphodiester bond between a dNTP, dNTP analogue, rNTP, or rNTP analogue and a primer or a DNA or RNA extension product.

In a further embodiment of the methods of the invention, said sensors of said array each occupy an area of 100 µm or less and have a pitch of 10 µm or less and wherein each of said reaction chambers has a volume in the range of from 1 µm³ to 1500 µm³; each of said reaction chambers contains at least 10⁵ copies of the single-stranded DNA or RNA in the solution; or said plurality of said reaction chambers and said plurality of said sensors are each greater in number than 256,000.

In a further embodiment of the methods of the invention, single-stranded DNA(s) or RNA(s) in the solution are attached to a solid substrate; wherein a primer in the solution is attached to a solid substrate; wherein the single-stranded DNA or RNA or primer is attached to a solid substrate via 1,3-dipolar azide-alkyne cycloaddition chemistry; the single-stranded DNA or RNA or primer is attached to a solid substrate via a polyethylene glycol molecule; wherein the single-stranded DNA or RNA or primer is attached to a solid substrate via a polyethylene glycol molecule and is azide-functionalized; the single-stranded DNA or RNA or primer is attached to a solid substrate via an azido linkage, an alkynyl linkage, or biotin-streptavidin interaction; the single-stranded DNA or RNA or primer is alkyne-labeled; the single-stranded DNA or RNA or primer is attached to a solid substrate which is in the form of a chip, a bead, a well, a capillary tube, a slide, a wafer, a filter, a fiber, a porous media, a matrix, a porous nanotube, or a column; wherein the single-stranded DNA or RNA or primer is attached to a solid substrate which is a metal, gold, silver, quartz, silica, a plastic, polypropylene, a glass, nylon, or diamond; the single-stranded DNA or RNA or primer is attached to a solid substrate which is a porous non-metal substance to which is attached or impregnated a metal or combination of metals; wherein the single-stranded DNA or RNA or primer is attached to a solid substrate which is in turn attached to a second solid substrate; or the single-stranded DNA or RNA or primer is attached to a solid substrate which is in turn attached to a second solid substrate which is a chip.

In a further embodiment of the methods of the invention, 1x10⁹ or fewer copies of the single-stranded DNA or RNA or primer are attached to a solid substrate; 1x10⁸ or fewer copies of the single-stranded DNA or RNA or primer are attached to a solid substrate; 2x10⁷ or fewer copies of the single-stranded DNA or RNA or primer are attached to a solid substrate; 1x10⁷ or fewer copies of the single-stranded DNA or RNA or primer are attached to a solid substrate; 1x10⁶ or fewer copies of the single-stranded DNA or RNA or primer are attached to a solid substrate; 1x10⁴ or fewer copies of the single-stranded DNA or RNA or primer are attached to a solid substrate; or 1,000 or fewer copies of the single-stranded DNA or RNA or primer are attached to a solid substrate.

In a further embodiment of the methods of the invention, 10,000 or more copies of the single-stranded DNA or RNA or primer are attached to a solid substrate; 1x10⁷ or more copies of the DNA or RNA or primer are attached to a solid substrate; 1x10⁸ or more copies of the single-stranded DNA or RNA or primer are attached to a solid substrate; 1x10⁹ or more copies of the single-stranded DNA or RNA or primer are attached to a solid substrate; or the single-stranded DNA or RNA or primer are separated in discrete compartments, wells, or depressions on a solid surface.

In a further embodiment of the methods of the invention, the method is performed in parallel on a plurality of single-stranded DNAs or RNAs; and wherein optionally the single-stranded DNAs or RNAs are templates having the same sequence.

In a further embodiment of the methods of the invention, the method further comprises, after the residue of the nucleotide residue has been determined in step (b), or (c), as appropriate, permanently capping any unextended primers or unextended DNA or RNA extension products.

In a further embodiment of the methods of the invention, the single-stranded DNA or RNA is amplified from a sample of DNA or RNA prior to step (a); and wherein optionally the single-stranded DNA or RNA is amplified by polymerase chain reaction.

The specification discloses a method for determining the identity of a nucleotide residue of a single-stranded DNA in a solution comprising:
(a) contacting the single-stranded DNA, having a primer hybridized to a portion thereof, with a DNA polymerase and a deoxyribonucleotide triphosphate (dNTP) analogue under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 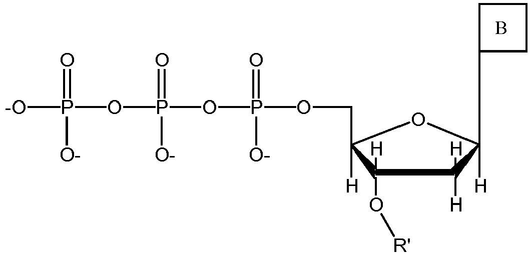 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the dNTP analogue into the primer to form a DNA extension product has occurred in step (a) by determining if an increase in hydrogen ion concentration of the solution has occured, wherein
   (i) if the dNTP analogue has been incorporated into the primer, determining from the identity of the incorporated dNTP analogue the identity of the nucleotide residue in the single-stranded DNA complementary thereto, thereby determining the identity of the nucleotide residue in the single-stranded DNA, and
   (ii) if no change in hydrogen ion concentration has occurred, iteratively performing step (a), wherein in each iteration of step (a) the dNTP analogue comprises a base which is a different type of base from the type of base of the dNTP analogues in every preceding iteration of step (a), until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA.

The specification discloses a method for determining the sequence of consecutive nucleotide residues in a single-stranded DNA in a solution comprising:
(a) contacting the single-stranded DNA, having a primer hybridized to a portion thereof, with a DNA polymerase and a deoxyribonucleotide triphosphate (dNTP) analogue under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 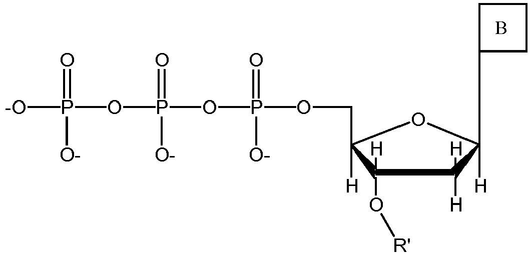 wherein B is a base and is adenine, guanine, cytosine, or thymine, and (2) R' is (i) -CH₂N₃, or 2-nitrobenzyl, or (ii) is a hydrocarbyl, or a substituted hydrocarbyl, having a mass of less than 300 daltons;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the primer to form a DNA extension product, and if so, determining from the identity of the incorporated dNTP analogue the identity of the nucleotide residue in the single-stranded DNA complementary thereto, thereby determining the identity of the nucleotide residue in the single-stranded DNA, and wherein no change in hydrogen ion concentration indicates that the dNTP analogue has not been incorporated into the primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA;
(d) if an increase in hydrogen ion concentration has been detected and a dNTP analogue is incorporated, subsequently treating the incorporated dNTP nucleotide analogue so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded DNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded DNA which is immediately 5' to a nucleotide residue of the single-stranded DNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded DNA so as to thereby determine the sequence of the consecutive nucleotide residues of the DNA.

In one embodiment of any of the inventions described herein, R' is -CH₂N₃.

In another embodiment of any of the inventions described herein, R' is a substituted hydrocarbyl, and is a nitrobenzyl. In a further embodiment, R' is a 2-nitrobenzyl.

In another embodiment of any of the inventions described herein, R' is a hydrocarbyl, and is allyl (-CH₂-CH=CH₂).

In one embodiment of any of the inventions described herein, the DNA is in a solution in a reaction chamber disposed on a sensor which is (i) formed in a semiconductor substrate and (ii) comprises a field-effect transistor or chemical field-effect transistor configured to provide at least one output signal in response to an increase in hydrogen ion concentration of the solution resulting from the formation of a phosphodiester bond between a nucleotide triphosphate or nucleotide triphosphate analogue and a primer or a DNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a field-effect transistor configured to provide at least one output signal in response to an increase in hydrogen ion concentration of the solution resulting from the formation of a phosphodiester bond between a nucleotide triphosphate or nucleotide triphosphate analogue and a primer or a DNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a chemical field-effect transistor configured to provide at least one output electrical signal in response to an increase in hydrogen ion concentration of the solution resulting from the formation of a phosphodiester bond between a nucleotide triphosphate or nucleotide triphosphate analogue and a primer or a DNA extension product. In another embodiment, said sensors of said array each occupy an area of 100 µm or less and have a pitch of 10 µm or less and wherein each of said reaction chambers has a volume in the range of from 1 µm³ to 1500 µm³. In another embodiment, each of said reaction chambers contains at least 10⁵ copies of the single-stranded DNA in the solution. In another embodiment, said plurality of said reaction chambers and said plurality of said sensors are each greater in number than 256,000.

In another embodiment of any of the inventions described herein, single-stranded DNA(s) in the solution are attached to a solid substrate. In another embodiment of any of the inventions described herein, a primer in the solution is attached to a solid substrate. In an embodiment, the single-stranded DNA or primer is attached to a solid substrate via a polyethylene glycol molecule. In a further embodiment, the solid substrate is azide-functionalized. In an embodiment, the DNA or primer is attached to a solid substrate via an azido linkage, an alkynyl linkage, or biotin-streptavidin interaction. In an embodiment, the DNA or primer is alkyne-labeled.

In another embodiment of any of the inventions described herein, the DNA or primer is attached to a solid substrate which is in the form of a chip, a bead, a well, a capillary tube, a slide, a wafer, a filter, a fiber, a porous media, a matrix, a porous nanotube, or a column. In another embodiment, the DNA or primer is attached to a solid substrate which is a metal, gold, silver, quartz, silica, a plastic, polypropylene, a glass, nylon, or diamond. In another embodiment, the DNA or primer is attached to a solid substrate which is a porous non-metal substance to which is attached or impregnated a metal or combination of metals. In another embodiment, the DNA or primer is attached to a solid substrate which is in turn attached to a second solid substrate. In a further embodiment, the second solid substrate is a chip.

In another embodiment of any of the inventions described herein, 1x10⁹ or fewer copies of the DNA or primer are attached to the solid substrate. In further embodiments, 1x10⁸ or fewer, 2x10⁷ or fewer, 1x10⁷ or fewer, 1x10⁶ or fewer, 1x10⁴ or fewer, or 1,000 or fewer copies of the DNA or primer are attached to the solid substrate.

In another embodiment of any of the inventions described herein, 10,000 or more copies of the DNA or primer are attached to the solid substrate. In further embodiments, 1x10⁷ or more, 1x10⁸ or more, or 1x10⁹ or more copies of the DNA or primer are attached to the solid substrate.

In another embodiment of any of the inventions described herein, the DNA or primer are separated in discrete compartments, wells, or depressions on a solid surface.

In another embodiment, in each dNTP analogue, R' has the structure: 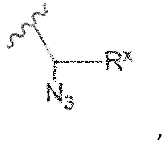 where R^{x} is, independently, a C₁-C₅ alkyl, a C₂-C₅ alkenyl, or a C₂-C₅ alkynyl, which is substituted or unsubstituted and which has a mass of less than 300 daltons or H, wherein the wavy line indicates the point of attachment to the 3' oxygen atom.

In another embodiment, in each dNTP analogue R' has the structure: 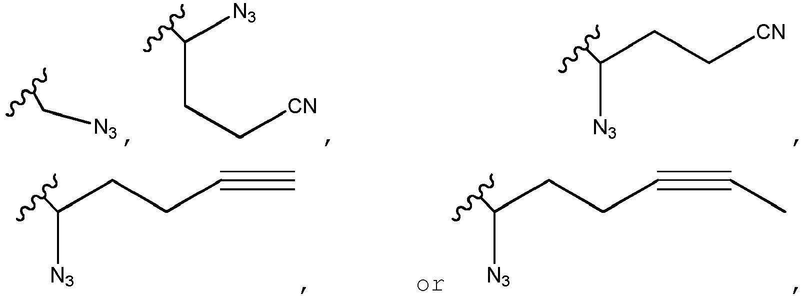 wherein the wavy line indicates the point of attachment to the 3' oxygen atom.

In one embodiment, the method is performed in parallel on a plurality of single-stranded DNAs. In another embodiment, the single-stranded DNAs are templates having the same sequence. In another embodiment, the method further comprises contacting the plurality of single-stranded DNAs or templates after the residue of the nucleotide residue has been determined in step (b), or (c), as appropriate, with a dideoxynucleotide triphosphate which is complementary to the nucleotide residue which has been identified, so as to thereby permanently cap any unextended primers or unextended DNA extension products.

In an embodiment of any of the methods described herein, the single-stranded DNA is amplified from a sample of DNA prior to step (a). In an embodiment of the methods described herein the single-stranded DNA is amplified by polymerase chain reaction.

In an embodiment of any of the inventions described herein, UV light is used to treat the R' group of a dNTP analogue incorporated into a primer or DNA extension product so as to photochemically cleave the moiety attached to the 3'-O so as to replace the 3'-O-R' with a 3'-OH. In a further embodiment, the moiety is a 2-nitrobenzyl moiety.

The specification discloses a method for determining the identity of a nucleotide residue of a single-stranded RNA in a solution comprising:
(a) contacting the single-stranded RNA, having an RNA primer hybridized to a portion thereof, with a polymerase and a ribonucleotide triphosphate (rNTP) analogue under conditions permitting the polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the primer, so as to form an RNA extension product, wherein (1) the rNTP analogue has the structure: 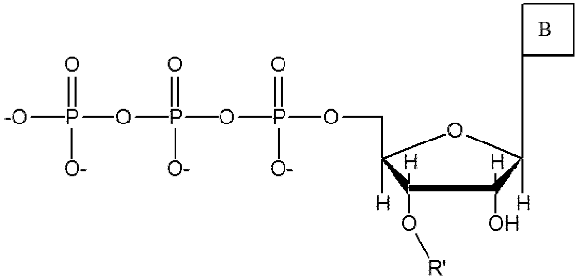 wherein B is a base and is adenine, guanine, cytosine, or uracil, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the rNTP analogue into the RNA primer to form an RNA extension product has occurred in step (a) by determining if an increase in hydrogen ion concentration of the solution has occured, wherein
   (i) if the rNTP analogue has been incorporated into the RNA primer, determining from the identity of the incorporated rNTP analogue the identity of the nucleotide residue in the single-stranded RNA complementary thereto, thereby determining the identity of the nucleotide residue in the single-stranded RNA, and
   (ii) if no change in hydrogen ion concentration has occurred, iteratively performing step (a), wherein in each iteration of step (a) the rNTP analogue comprises a base which is a different type of base from the type of base of the rNTP analogues in every preceding iteration of step (a), until an rNTP analogue is incorporated into the RNA primer to form an RNA extension product, and determining from the identity of the incorporated rNTP analogue the identity of the nucleotide residue in the single-stranded RNA complementary thereto, thereby determining the identity of the nucleotide residue in the single-stranded RNA.

The specification discloses a method for determining the sequence of consecutive nucleotide residues in a single-stranded RNA in a solution comprising:
(a) contacting the single-stranded RNA, having an RNA primer hybridized to a portion thereof, with a polymerase and a ribonucleotide triphosphate (rNTP) analogue under conditions permitting the polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the RNA primer, so as to form an RNA extension product, wherein (1) the rNTP analogue has the structure: 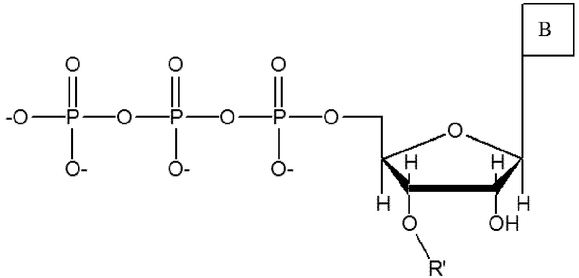 wherein B is a base and is adenine, guanine, cytosine, or uracil, and (2) R' is (i)-CH₂N₃, or 2-nitrobenzyl, or (ii) is a hydrocarbyl, or a substituted hydrocarbyl, having a mass of less than 300 daltons;
(b) determining whether incorporation of the rNTP analogue has occurred in step (a) by detecting an increase in hydrogen ion concentration of the solution, wherein an increase in hydrogen ion concentration indicates that the rNTP analogue has been incorporated into the RNA primer to form an RNA extension product, and if so, determining from the identity of the incorporated rNTP analogue the identity of the nucleotide residue in the single-stranded RNA complementary thereto, thereby determining the identity of the nucleotide residue in the single-stranded RNA, and wherein no change in hydrogen ion concentration indicates that the rNTP analogue has not been incorporated into the RNA primer in step (a);
(c) if no change in hydrogen ion concentration has been detected in step (b), iteratively performing steps (a) and (b), wherein in each iteration of step (a) for a given nucleotide residue, the identity of which is being determined, the rNTP analogue comprises a base which is a different type of base from the type of base of the rNTP analogues in every preceding iteration of step (a) for that nucleotide residue, until an rNTP analogue is incorporated into the primer to form an RNA extension product, and determining from the identity of the incorporated rNTP analogue the identity of the nucleotide residue in the single-stranded RNA complementary thereto, thereby determining the identity of the nucleotide residue in the single-stranded RNA;
(d) if an increase in hydrogen ion concentration has been detected and an rNTP analogue is incorporated, subsequently treating the incorporated rNTP nucleotide analogue so as to replace the R' group thereof with an H atom thereby providing a 3' OH group at the 3' terminal of the RNA extension product; and
(e) iteratively performing steps (a) to (d), as necessary, for each nucleotide residue of the consecutive nucleotide residues of the single-stranded RNA to be sequenced, except that in each repeat of step (a) the rNTP analogue is (i) incorporated into the RNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the RNA extension product resulting from a preceding iteration of step (a) or step (c), so as to form a subsequent RNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

In one embodiment of any of the inventions described herein, R' is -CH₂N₃.

In another embodiment of any of the inventions described herein, R' is a substituted hydrocarbyl, and is a nitrobenzyl. In a further embodiment, R' is a 2-nitrobenzyl.

In another embodiment of any of the inventions described herein, R' is a hydrocarbyl, and is allyl (-CH₂-CH=CH₂).

In one embodiment of any of the inventions described herein, the RNA is in a solution in a reaction chamber disposed on a sensor which is (i) formed in a semiconductor substrate and (ii) comprises a field-effect transistor or chemical field-effect transistor configured to provide at least one output signal in response to an increase in hydrogen ion concentration of the solution resulting from the formation of a phosphodiester bond between a nucleotide triphosphate or nucleotide triphosphate analogue and a primer or an RNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a field-effect transistor configured to provide at least one output signal in response to an increase in hydrogen ion concentration of the solution resulting from the formation of a phosphodiester bond between a nucleotide triphosphate or nucleotide triphosphate analogue and a primer or an RNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a chemical field-effect transistor configured to provide at least one output electrical signal in response to an increase in hydrogen ion concentration of the solution resulting from the formation of a phosphodiester bond between a nucleotide triphosphate or nucleotide triphosphate analogue and a primer or an RNA extension product. In another embodiment, said sensors of said array each occupy an area of 100 µm or less and have a pitch of 10 µm or less and wherein each of said reaction chambers has a volume in the range of from 1 µm³ to 1500 µm³. In another embodiment, each of said reaction chambers contains at least 10⁵ copies of the single-stranded RNA in the solution. In another embodiment, said plurality of said reaction chambers and said plurality of said sensors are each greater in number than 256,000.

In another embodiment of any of the inventions described herein, single-stranded RNA(s) in the solution are attached to a solid substrate. In another embodiment of any of the inventions described herein, a primer in the solution is attached to a solid substrate. In an embodiment, the single-stranded RNA or primer is attached to a solid substrate via a polyethylene glycol molecule. In a further embodiment, the solid substrate is azide-functionalized. In an embodiment, the RNA or primer is attached to a solid substrate via an azido linkage, an alkynyl linkage, or biotin-streptavidin interaction. In an embodiment, the RNA or primer is alkyne-labeled.

In another embodiment of any of the inventions described herein, the RNA or primer is attached to a solid substrate which is in the form of a chip, a bead, a well, a capillary tube, a slide, a wafer, a filter, a fiber, a porous media, a matrix, a porous nanotube, or a column. In another embodiment, the RNA or primer is attached to a solid substrate which is a metal, gold, silver, quartz, silica, a plastic, polypropylene, a glass, nylon, or diamond. In another embodiment, the RNA or primer is attached to a solid substrate which is a porous non-metal substance to which is attached or impregnated a metal or combination of metals. In another embodiment, the RNA or primer is attached to a solid substrate which is in turn attached to a second solid substrate. In a further embodiment, the second solid substrate is a chip.

In another embodiment of any of the inventions described herein, 1x10⁹ or fewer copies of the RNA or primer are attached to the solid substrate. In further embodiments, 1x10⁸ or fewer, 2x10⁷ or fewer, 1x10⁷ or fewer, 1x10⁶ or fewer, 1x10⁴ or fewer, or 1,000 or fewer copies of the RNA or primer are attached to the solid substrate.

In another embodiment of any of the inventions described herein, 10,000 or more copies of the RNA or primer are attached to the solid substrate. In further embodiments, 1x10⁷ or more, 1x10⁸ or more, or 1x10⁹ or more copies of the RNA or primer are attached to the solid substrate.

In another embodiment of any of the inventions described herein, the RNA or primer are separated in discrete compartments, wells, or depressions on a solid surface.

In another embodiment, in each rNTP analogue, R' has the structure: 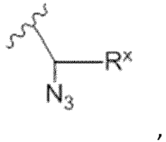 where R^{x} is, independently, a C₁-C₅ alkyl, a C₂-C₅ alkenyl, or a C₂-C₅ alkynyl, which is substituted or unsubstituted and which has a mass of less than 300 daltons, or H, wherein the wavy line indicates the point of attachment to the 3' oxygen atom.

In another embodiment, the rNTP analogue R' has the structure: 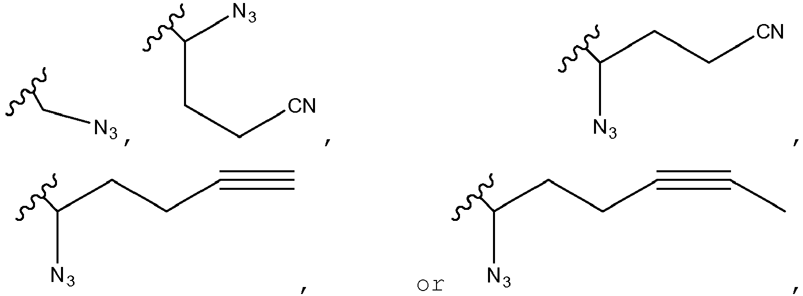 wherein the wavy line indicates the point of attachment to the 3' oxygen atom.

In one embodiment, the method is performed in parallel on a plurality of RNAs. In another embodiment, the RNAs are templates having the same sequence. In another embodiment, the method further comprises contacting the plurality of RNAs or templates after the residue of the nucleotide residue has been determined in step (b), or (c), as appropriate, with a dinucleotide triphosphate which is complementary to the nucleotide residue which has been identified, so as to thereby permanently cap any unextended primers or unextended RNA extension products.

In an embodiment of any of the methods described herein, the single-stranded RNA is amplified from a sample of RNA prior to step (a). In a further embodiment the single-stranded RNA is amplified by reverse transcriptase polymerase chain reaction.

In an embodiment of any of the inventions described herein, UV light is used to treat the R' group of an rNTP analogue incorporated into a primer or RNA extension product so as to photochemically cleave the moiety attached to the 3'-O so as to replace the 3'-O-R' with a 3'-OH. In a further embodiment, the moiety is a 2-nitrobenzyl moiety.

The specification discloses a method for determining the identity of a nucleotide residue of a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the DNA primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 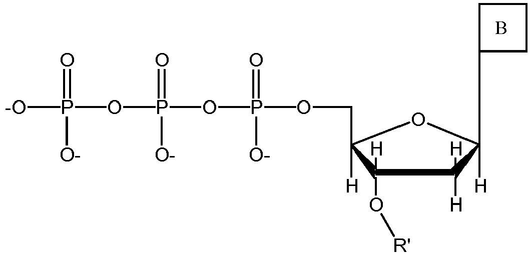 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons; and
(b) determining whether incorporation of the dNTP analogue into the DNA primer to form a DNA extension product has occurred in step (a) by determining if an increase in hydrogen ion concentration of the solution has occured, wherein
   (i) if the dNTP analogue has been incorporated into the DNA primer, determining from the identity of the incorporated dNTP analogue the identity of the nucleotide residue in the single-stranded RNA complementary thereto, thereby determining the identity of the nucleotide residue in the single-stranded RNA, and
   (ii) if no change in hydrogen ion concentration has occurred, iteratively performing step (a), wherein in each iteration of step (a) the dNTP analogue comprises a base which is a different type of base from the type of base of the dNTP analogues in every preceding iteration of step (a), until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA.

The specification discloses a method for determining the sequence of consecutive nucleotide residues in a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 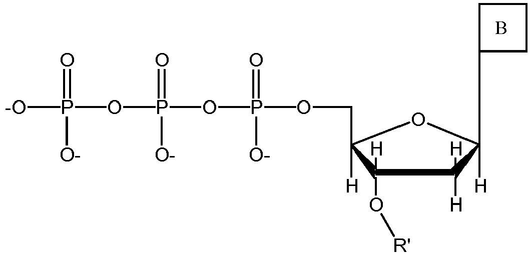 wherein B is a base and is adenine, guanine, cytosine, or thymine, and (2) R' is (i) -CH₂N₃ or 2-nitrobenzyl, or (ii) is a hydrocarbyl, or a substituted hydrocarbyl, having a mass of less than 300 daltons;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the DNA primer to form a DNA extension product, and if so, determining from the identity of the incorporated dNTP analogue the identity of the nucleotide residue in the single-stranded RNA complementary thereto, thereby determining the identity of the nucleotide residue in the single-stranded RNA, and wherein no change in hydrogen ion concentration indicates that the dNTP analogue has not been incorporated into the DNA primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA;
(d) if an increase in hydrogen ion concentration has been detected and a dNTP analogue is incorporated, subsequently treating the incorporated dNTP nucleotide analogue so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded RNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
   thereby determining the identity of each of the consecutive nucleotide residues of the single-stranded RNA so as to thereby determine the sequence of the consecutive nucleotide residues of the RNA.

In one embodiment of any of the inventions described herein, R' is -CH₂N₃.

In another embodiment of any of the inventions described herein, R' is a substituted hydrocarbyl, and is a nitrobenzyl. In a further embodiment, R' is a 2-nitrobenzyl.

In another embodiment of any of the inventions described herein, R' is a hydrocarbyl, and is allyl (-CH₂-CH=CH₂).

In one embodiment of any of the inventions described herein, the RNA is in a solution in a reaction chamber disposed on a sensor which is (i) formed in a semiconductor substrate and (ii) comprises a field-effect transistor or chemical field-effect transistor configured to provide at least one output signal in response to an increase in hydrogen ion concentration of the solution resulting from the formation of a phosphodiester bond between a nucleotide triphosphate or nucleotide triphosphate analogue and a primer or a DNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a field-effect transistor configured to provide at least one output signal in response to an increase in hydrogen ion concentration of the solution resulting from the formation of a phosphodiester bond between a nucleotide triphosphate or nucleotide triphosphate analogue and a primer or a DNA extension product.

In another embodiment of any of the inventions described herein, the reaction chamber is one of a plurality of reaction chambers disposed on a sensor array formed in a semiconductor substrate and comprised of a plurality of sensors, each reaction chamber being disposed on at least one sensor and each sensor of the array comprising a chemical field-effect transistor configured to provide at least one output electrical signal in response to an increase in hydrogen ion concentration of the solution resulting from the formation of a phosphodiester bond between a nucleotide triphosphate or nucleotide triphosphate analogue and a primer or a DNA extension product. In another embodiment, said sensors of said array each occupy an area of 100 µm or less and have a pitch of 10 µm or less and wherein each of said reaction chambers has a volume in the range of from 1 µm³ to 1500 µm³. In another embodiment, each of said reaction chambers contains at least 10⁵ copies of the single-stranded RNA in the solution. In another embodiment, said plurality of said reaction chambers and said plurality of said sensors are each greater in number than 256,000.

In another embodiment of any of the inventions described herein, single-stranded RNA(s) in the solution are attached to a solid substrate. In an embodiment, the single-stranded RNA or primer is attached to a solid substrate via a polyethylene glycol molecule. In a further embodiment, the solid substrate is azide-functionalized. In an embodiment, the RNA or primer is attached to a solid substrate via an azido linkage, an alkynyl linkage, or biotin-streptavidin interaction. In an embodiment, the RNA or primer is alkyne-labeled.

In another embodiment of any of the inventions described herein, the RNA or primer is attached to a solid substrate which is in the form of a chip, a bead, a well, a capillary tube, a slide, a wafer, a filter, a fiber, a porous media, a matrix, a porous nanotube, or a column. In another embodiment, the RNA or primer is attached to a solid substrate which is a metal, gold, silver, quartz, silica, a plastic, polypropylene, a glass, nylon, or diamond. In another embodiment, the RNA or primer is attached to a solid substrate which is a porous non-metal substance to which is attached or impregnated a metal or combination of metals. In another embodiment, the RNA or primer is attached to a solid substrate which is in turn attached to a second solid substrate. In a further embodiment, the second solid substrate is a chip.

In another embodiment of any of the inventions described herein, 1x10⁹ or fewer copies of the RNA or primer are attached to the solid substrate. In further embodiments, 1x10⁸ or fewer, 2x10⁷ or fewer, 1x10⁷ or fewer, 1x10⁶ or fewer, 1x10⁴ or fewer, or 1,000 or fewer copies of the RNA or primer are attached to the solid substrate.

In another embodiment of any of the inventions described herein, 10,000 or more copies of the RNA or primer are attached to the solid substrate. In further embodiments, 1x10⁷ or more, 1x10⁸ or more, or 1x10⁹ or more copies of the RNA or primer are attached to the solid substrate.

In another embodiment of any of the inventions described herein, the RNA or primer are separated in discrete compartments, wells, or depressions on a solid surface.

In another embodiment, in each dNTP analogue, R' has the structure: 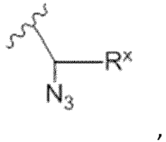 where R^{x} is, independently, a C₁-C₅ alkyl, a C₂-C₅ alkenyl, or a C₂-C₅ alkynyl, which is substituted or unsubstituted and which has a mass of less than 300 daltons.

In another embodiment, in each dNTP analogue, R' has the structure: 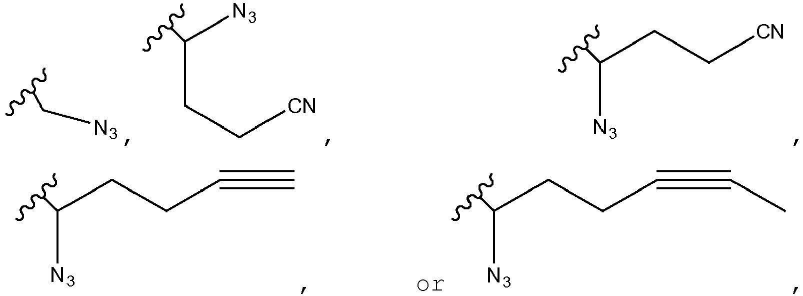 wherein the wavy line indicates the point of attachment to the 3' oxygen atom.

In one embodiment, the method is performed in parallel on a plurality of single-stranded RNAs. In another embodiment, the single-stranded RNAs are templates having the same sequence. In another embodiment, the method further comprises contacting the plurality of single-stranded RNAs or templates after the residue of the nucleotide residue has been determined in step (b), or (c), as appropriate, with a dideoxynucleotide triphosphate which is complementary to the nucleotide residue which has been identified, so as to thereby permanently cap any unextended primers or unextended DNA extension products.

In an embodiment of any of the methods described herein, the single-stranded RNA is amplified from a sample of RNA prior to step (a). In a further embodiment the single-stranded RNA is amplified by reverse transcriptase polymerase chain reaction.

In an embodiment of any of the inventions described herein, UV light is used to treat the R' group of a dNTP analogue incorporated into a primer or DNA extension product so as to photochemically cleave the moiety attached to the 3'-O so as to replace the 3'-O-R' with a 3'-OH. In a further embodiment, the moiety is a 2-nitrobenzyl moiety.

Examples of attaching nucleic acids to solid substrates, or immobilization of nucleic acids, are described in Immobilization of DNA on Chips II, edited by Christine Wittmann (2005), Springer Verlag, Berlin.

Ion sensitive field effect transistors (FET) and methods and apparatus for measuring H⁺ generated by sequencing by synthesis reactions using large scale FET arrays are known in the art and described in U.S. Patent Application Publication Nos. US 20100035252, US 20100137143, US 20100188073, US 20100197507, US 20090026082, US 20090127589, US 20100282617, US 20100159461, US20080265985, US 20100151479, US 20100255595, U.S. Patents 7,686,929 and 7,649,358, and PCT International Publication Nos. WO/2009/158006 A3, WO/2008/076406 A2, WO/2010/008480 A2, WO/2010/008480 A3, WO/2010/016937 A2, WO/2010/047804 A1, and WO/2010/016937 A3.

As used herein, "hydrocarbon" refers to a compound containing hydrogen and carbon. A "hydrocarbyl" refers to a hydrocarbon which has had one hydrogen removed. Hydrocarbyls may be unsubstituted or substituted. For example, hydrocarbyls may include alkyls (such as methyl or ethyl), alkenyls (such as ethenyl and propenyl), alkynyls (such as ethynyl and propynyl), and phenyls (such as benzyl).

As used herein, "alkyl" includes both branched and straight-chain saturated aliphatic hydrocarbon groups having the specified number of carbon atoms and may be unsubstituted or substituted. Thus, C₁-Cn as in "C₁-Cn alkyl" is defined to include groups having 1, 2, ...., n-1 or n carbons in a linear or branched arrangement. For example, a "C₁-C₅ alkyl" is defined to include groups having 1, 2, 3, 4, or 5 carbons in a linear or branched arrangement, and specifically includes methyl, ethyl, n-propyl, isopropyl, n-butyl, t-butyl, and pentyl.

As used herein, "alkenyl" refers to a non-aromatic hydrocarbon radical, straight or branched, containing at least 1 carbon to carbon double bond, and up to the maximum possible number of non-aromatic carbon-carbon double bonds may be present, and may be unsubstituted or substituted. For example, "C₂-C₅ alkenyl" means an alkenyl radical having 2, 3, 4, or 5, carbon atoms, and up to 1, 2, 3, or 4, carbon-carbon double bonds respectively. Alkenyl groups include ethenyl, propenyl, and butenyl.

As used herein, "alkynyl" refers to a hydrocarbon radical straight or branched, containing at least 1 carbon to carbon triple bond, and up to the maximum possible number of non-aromatic carbon-carbon triple bonds may be present, and may be unsubstituted or substituted. Thus, "C₂-C₅ alkynyl" means an alkynyl radical having 2 or 3 carbon atoms and 1 carbon-carbon triple bond, or having 4 or 5 carbon atoms and up to 2 carbon-carbon triple bonds. Alkynyl groups include ethynyl, propynyl and butynyl.

As used herein, "substituted" refers to a functional group as described above such as an alkyl, or a hydrocarbyl, in which at least one bond to a hydrogen atom contained therein is replaced by a bond to non-hydrogen or non-carbon atom, provided that normal valencies are maintained and that the substitution(s) result(s) in a stable compound. Substituted groups also include groups in which one or more bonds to a carbon(s) or hydrogen(s) atom are replaced by one or more bonds, including double or triple bonds, to a heteroatom. Non-limiting examples of substituents include the functional groups described above, -NO₂, and, for example, N, e.g. so as to form -CN.

As used herein, and unless stated otherwise, each of the following terms shall have the definition set forth below.
- A: - Adenine;
- C: - Cytosine;
- DNA: - Deoxyribonucleic acid;
- G: - Guanine;
- RNA: - Ribonucleic acid;
- T: - Thymine;
- U: - Uracil; and
- NRT: - Nucleotide Reversible Terminator.

"Nucleic acid" shall mean, unless otherwise specified, any nucleic acid molecule, including, without limitation, DNA, RNA and hybrids thereof. In an embodiment the nucleic acid bases that form nucleic acid molecules can be the bases A, C, G, T and U, as well as derivatives thereof. Derivatives of these bases are well known in the art, and are exemplified in PCR Systems, Reagents and Consumables (Perkin Elmer Catalogue 1996-1997, Roche Molecular Systems, Inc., Branchburg, New Jersey, USA). In an embodiment the DNA or RNA is not modified. In an embodiment the DNA or RNA is modified only insofar as it is attached to a surface, such as a solid surface.

"Solid substrate" or "solid support" shall mean any suitable medium present in the solid phase to which a nucleic acid or an agent may be affixed. Non-limiting examples include chips, beads, nanopore structures and columns. In an embodiment the solid substrate or solid support can be present in a solution, including an aqueous solution, a gel, or a fluid.

"Hybridize" shall mean the annealing of one single-stranded nucleic acid to another nucleic acid based on the well-understood principle of sequence complementarity. In an embodiment the other nucleic acid is a single-stranded nucleic acid. The propensity for hybridization between nucleic acids depends on the temperature and ionic strength of their milieu, the length of the nucleic acids and the degree of complementarity. The effect of these parameters on hybridization is well known in the art (see Sambrook J, Fritsch EF, Maniatis T. 1989. Molecular cloning: a laboratory manual. Cold Spring Harbor Laboratory Press, New York.). As used herein, hybridization of a primer sequence, or of a DNA extension product, to another nucleic acid shall mean annealing sufficient such that the primer, or DNA extension product, respectively, is extendable by creation of a phosphodiester bond with an available nucleotide or nucleotide analogue capable of forming a phosphodiester bond.

As used herein, unless otherwise specified, a base of a nucleotide or nucleotide analogue which is a "different type of base from the type of base" (of a reference) means the base has a different chemical structure from the other/reference base or bases. For example, a base that is "different from" adenine would include a base that is guanine, a base that is uracil, a base that is cytosine, and a base that is thymine. For example, a base that is "different from" adenine, thymine, and cytosine would include a base that is guanine and a base that is uracil.

As used herein, "primer" (a primer sequence) is a short, often chemically synthesized, oligonucleotide of appropriate length, for example about 18-24 bases, sufficient to hybridize to a target nucleic acid (e.g. a single-stranded nucleic acid) and permit the addition of a nucleotide residue thereto, or oligonucleotide or polynucleotide synthesis therefrom, under suitable conditions well-known in the art. The target nucleic acid may be self-priming. In an embodiment the primer is a DNA primer, i.e. a primer consisting of, or largely consisting of deoxyribonucleotide residues. In another embodiment the primer is an RNA primer, i.e. a primer consisting of, or largely consisting of ribonucleotide residues. The primers are designed to have a sequence which is the reverse complement of a region of template/target DNA or RNA to which the primer hybridizes. The addition of a nucleotide residue to the 3' end of a DNA primer by formation of a phosphodiester bond results in the primer becoming a "DNA extension product." The addition of a nucleotide residue to the 3' end of the DNA extension product by formation of a phosphodiester bond results in a further DNA extension product. The addition of a nucleotide residue to the 3' end of an RNA primer by formation of a phosphodiester bond results in the primer becoming an "RNA extension product." The addition of a nucleotide residue to the 3' end of the RNA extension product by formation of a phosphodiester bond results in a further RNA extension product. A "probe" is a primer with a detectable label or attachment.

As used herein a nucleic acid, such as a single-stranded DNA or RNA, "in a solution" means the nucleic acid is submerged in an appropriate solution. The nucleic acid in the solution may be attached to a surface, including a solid surface. Thus, as used herein, "in a solution", unless context indicates otherwise, encompasses, for example, both a DNA free in a solution and a DNA in a solution wherein the DNA is tethered to a solid surface.

A "nucleotide residue" is a single nucleotide in the state it exists after being incorporated into, and thereby becoming a monomer of, a polynucleotide. Thus, a nucleotide residue is a nucleotide monomer of a polynucleotide, e.g. DNA, which is bound to an adjacent nucleotide monomer of the polynucleotide through a phosphodiester bond at the 3' position of its sugar and is bound to a second adjacent nucleotide monomer through its phosphate group, with the exceptions that (i) a 3' terminal nucleotide residue is only bound to one adjacent nucleotide monomer of the polynucleotide by a phosphodiester bond from its phosphate group, and (ii) a 5' terminal nucleotide residue is only bound to one adjacent nucleotide monomer of the polynucleotide by a phosphodiester bond from the 3' position of its sugar.

Because of well-understood base-pairing rules, determination of which dNTP or rNTP analogue is incorporated into a primer or DNA or RNA extension product thereby reveals the identity of the complementary nucleotide residue in the single-stranded polynucleotide that the primer or DNA or RNA extension product is hybridized to. Thus, if the dNTP analogue that was incorporated comprises an adenine, a thymine, a cytosine, or a guanine, then the complementary nucleotide residue in the single-stranded DNA is identified as a thymine, an adenine, a guanine or a cytosine, respectively. The purine adenine (A) pairs with the pyrimidine thymine (T). The pyrimidine cytosine (C) pairs with the purine guanine (G). Similarly, with regard to RNA, where the RNA is hybridized to an RNA primer, if the rNTP analogue that was incorporated comprises an adenine, a uracil, a cytosine, or a guanine, then the complementary nucleotide residue in the single-stranded RNA is identified as a uracil, an adenine, a guanine or a cytosine, respectively. Where the RNA is hybridized to a DNA primer, if the dNTP analogue that was incorporated comprises an adenine, a thymine, a cytosine, or a guanine, then the complementary nucleotide residue in the single-stranded RNA is identified as a uracil, an adenine, a guanine or a cytosine, respectively.

Incorporation into an oligonucleotide or polynucleotide (such as a primer or DNA or RNA extension strand) of a dNTP or rNTP analogue means the formation of a phosphodiester bond between the 3' carbon atom of the 3' terminal nucleotide residue of the polynucleotide and the 5' carbon atom of the dNTP or rNTP analogue resulting in the loss of pyrophosphate from the dNTP or rNTP analogue.

As used herein, a deoxyribonucleotide triphosphate (dNTP) analogue, unless otherwise indicated, is a dNTP having substituted in the 3'-OH group of the sugar thereof, in place of the H atom of the 3'-OH group, or connected via a linker to the base thereof, a chemical group which is -CH₂N₃, or is a hydrocarbyl, or a substituted hydrocarbyl, having a mass of less than 300 daltons, and which does not prevent the dNTP analogue from being incorporated into a polynucleotide, such as DNA, by formation of a phosphodiester bond. Similarly, a deoxyribonucleotide analogue residue is a deoxyribonucleotide analogue which has been incorporated into a polynucleotide and which still comprises its chemical group which is -CH₂N₃, or is a hydrocarbyl, or a substituted hydrocarbyl, having a mass of less than 300 daltons. In a preferred embodiment of the deoxyribonucleotide triphosphate analogue, the chemical group is substituted in the 3'-OH group of the sugar thereof, in place of the H atom of the 3'-OH group. In a preferred embodiment of the deoxyribonucleotide analogue residue, the chemical group is substituted in the 3'-OH group of the sugar thereof, in place of the H atom of the 3'-OH group. In an embodiment the chemical group is -CH₂N₃.

As used herein, a ribonucleotide triphosphate (rNTP) analogue, unless otherwise indicated, is a rNTP having substituted in the 3'-OH group of the sugar thereof, in place of the H atom of the 3'-OH group, or connected via a linker to the base thereof, a chemical group which is -CH₂N₃, or is a hydrocarbyl, or a substituted hydrocarbyl, having a mass of less than 300 daltons, and which does not prevent the rNTP analogue from being incorporated into a polynucleotide, such as RNA, by formation of a phosphodiester bond. Similarly, a ribonucleotide analogue residue is a ribonucleotide analogue which has been incorporated into a polynucleotide and which still comprises its chemical group which is -CH₂N₃, or is a hydrocarbyl, or a substituted hydrocarbyl, having a mass of less than 300 daltons. In a preferred embodiment of the ribonucleotide triphosphate analogue, the chemical group is substituted in the 3'-OH group of the sugar thereof, in place of the H atom of the 3'-OH group. In a preferred embodiment of the ribonucleotide analogue residue, the chemical group is substituted in the 3'-OH group of the sugar thereof, in place of the H atom of the 3'-OH group. In an embodiment the chemical group is -CH₂N₃.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results.

In choosing the compounds of the present invention, one of ordinary skill in the art will recognize that the various substituents, i.e. R₁, Rₓ, etc. are to be chosen in conformity with well-known principles of chemical structure connectivity.

It is understood that where radicals are represented herein by structure, the point of attachment to the main structure is represented by a wavy line.

In the compound structures depicted herein, hydrogen atoms, except on ribose and deoxyribose sugars, are generally not shown. However, it is understood that sufficient hydrogen atoms exist on the represented carbon atoms to satisfy the octet rule.

Where a range of values is provided, unless the context clearly dictates otherwise, it is understood that each intervening integer of the value, and each tenth of each intervening integer of the value, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding (i) either or (ii) both of those included limits are also included in the invention.

### Experimental Details

There are a number of innovative aspects to the present invention. For example, the combination of the ion sensing strategy and the sequencing-by-synthesis approach using NRTs (Ju et al. 2003; Li et al. 2003; Ruparel et al. 2005; Seo et al. 2005; Ju et al. 2006) is a novel use of disparate sequencing paradigms to produce a hybrid approach that is very low cost, has good sensitivity, avoids false positive signals caused by spontaneous NTP depyrophosphorylation, and at the same time is as accurate as any of the available sequencing strategies.

Here it is disclosed that NRTs can be exploited for ion sensing SBS because: (1) NRTs display specificity and good processivity in polymerase extension; (2) NRTs permit the ion-sensing step to address single base incorporation, overcoming the complications of multiple base incorporation in homopolymer runs of different lengths; (3) synthesis of several alternative sets of NRTs with assorted blocking groups on the 3'-OH and elsewhere in the deoxyribose allows selection of the best NRTs with regard to speed and specificity of incorporation and ease of removal of the blocking group, while maintaining compatibility with DNA stability and ion sensing requirements (Li et al. 2003; Ruparel et al. 2005; Seo et al. 2005; Ju et al. 2006); (4) NRTs provide modified nucleotides that are identical to normal nucleotides after blocking group cleavage, thus allowing longer reads to be achieved; and (5) absence of fluorescent tags on the modified nucleotides increases polymerase incorporation efficiency, greatly lowering the cost of their synthesis, and removing the need to account for background fluorescence.

In the past, high-throughput DNA sequencing was accomplished by taking advantage of the automation possibilities afforded by the Sanger sequencing approach, relative to the competing chemical sequencing strategy (Sanger et al. 1977). Although use of 4-color fluorescent tags and capillary instruments enabled quite high throughput (Ju et al. 1995; Smith et al. 1986), up to >600-base reads every couple of hours per instrument, the DNA preparation procedures needed for whole genome sequencing were economically prohibitive, often necessitating DNA cloning and clone storage. Recent strategies utilizing either sequencing by synthesis (Roche pyrosequencing and Illumina instruments) or sequencing by hybridization and ligation (ABI's SOLID™ platform) have overcome this obstacle by taking advantage of variations on polony PCR (on beads or directly on sequencing chips) (Wheeler et al. 2008; Bentley et al. 2008; McKernan et al. 2009), and at the same time taken advantage of miniaturization strategies to allow millions of reads at the same time, dwarfing essentially all the advantages of the Sanger approach except its ability to generate fairly long reads. Still newer strategies endorsed by Helicos and Pacific Sciences have approached single-molecule sequencing, though at some cost to accuracy (Harris et al. 2008; Eid et al. 2008). Other options such as the use of nanopores to discriminate released nucleotides or the sequence of intact DNA chains are still being assessed (Branton et al. 2008).

For the sequencing by synthesis strategies, there are two general schemes that depend on the nature of the detection strategy. With detection of a single signal (light, a fluorescent dye, or a pH change in the case of Roche 454, Helicos, and Ion Torrent, respectively) upon the incorporation of each nucleotide, it is necessary to add each base one by one, and score the incorporation based on whether an output signal was generated. Such methods can reduce reagent cost and simplify the instrument design, but have lower overall accuracy. In contrast, methods that utilize multiple output signals (e.g. 4 fluorescent dyes, one for each of the bases of DNA), while involving more expensive reagents, can increase accuracy, particularly if background signals are reduced or computationally subtracted. Several of these methods, especially those of the first design, utilize standard dNTPs for incorporation and measure byproducts of the formation of the phosphodiester bond. A downside of this approach is difficulty in interpreting signals in homopolymer stretches. Even if only one of the dNTPs is added at a time, one must take into account the fact that if its complementary base is present at the next several positions, it would be important but difficult to determine exactly how many of the nucleotides were added in a row. The current protocols usually take additive measures of the signal, but beyond about 3 or 4 bases, it becomes difficult to distinguish base counts.

Here, it is disclosed that the use of 3'-O-modified nucleotide reversible terminators (NRTs) overcomes these problems.

*Ion sensing during sequencing by synthesis:* Recently, Ion Torrent, Inc. has introduced a sequencing method that leverages the enormous progress in the semiconductor field over the past decades. The method is based on the release of a H⁺ ion upon creation of the phosphodiester bond in the polymerase reaction. Reactions take place in a series of wells built into a chip, and a detection layer is attached to a semiconductor chip to *directly* convert the resulting pH change, a chemical signal, into digital data. This technology is rapid, inexpensive, highly scalable, and uses natural nucleotides. Because there is a single signal regardless of the nucleotide that gets incorporated, it is necessary to add the four nucleotides one at a time. This can lead to difficulty in interpreting signals in homopolymer stretches, places where a nucleotide will be incorporated multiple times in the same round of the reaction. This problem is solved herein by using specific NRTs, which have been successfully used as outlined hereinbelow.

*Sequencing by synthesis with reversible terminators:* A series of nucleotide reversible terminators (NRTs) to accomplish sequencing by synthesis has been described in numerous publications (Ju et al. 2006; Wu et al. 2007; Guo et al. 2008). In essence this process involves the use of nucleotide analogues that have blocking groups at the 3'-OH position, which, once incorporated into DNA, prevent addition of the subsequent nucleotide. DNA templates are bound to a surface and primers are hybridized to these templates. One can then measure the incorporation of a particular NRT onto the priming strand, due to its complementarity to a nucleotide on the template strand, by virtue of specific fluorophores attached to each base. These blocking groups and fluorophores can be easily removed using chemical or photo-cleavage reactions that do not damage the DNA template or primer. In this way, additional rounds of incorporation, detection and cleavage can take place. These SBS reactions are accurate, show no dephasing (reading ahead or lagging), and have relatively low background due to misincorporated nucleotides or incomplete removal of dyes.

Three different sets of 4 NRTs (**Fig. 1**), bearing either an allyl, azidomethyl, or 2-nitrobenzyl group at the 3'-OH position, were synthesized and used to conduct pyrosequencing. While the 2-nitrobenzyl group could be cleaved by light (∼355nm irradiation), simple chemicals were required to remove the allyl group (Na₂PdCl₄ plus trisodium triphenylphosphinetrisulfonate) or the azidomethyl group (Tris(2-carboxyethyl) phosphine) (Ju et al. 2006; Wu et al. 2007; Guo et al. 2008). Pyrosequencing was accomplished using each of these NRTs. Templates containing homopolymeric regions were immobilized on Sepharose beads, and extension-signal detection-deprotection cycles were conducted using the NRTs. As an example, pyrosequencing data using the NRTs modified by the photocleavable 2-nitrobenzyl group are shown in **Fig. 2****,** and compared with conventional pyrosequencing using natural nucleotides. As can be seen, multiple-base signals that could not be easily discriminated by conventional pyrosequencing were easily resolved using the NRTs.

It is disclosed here that 3'-O-(2-nitrobenzyl) nucleotides are particularly useful for ion sensor measurement. They are quickly and efficiently incorporated, and photo-cleaved under conditions that do not require the presence of salts which could interfere with subsequent rounds of ion sensing. However, other modified bases are also useful. The 3'-O-azidomethyl group is particularly attractive. Not only is it efficiently incorporated, but it regenerates the natural base upon cleavage, thus does not impede subsequent nucleotide incorporation, resulting in long sequence reads (Guo et al. 2008).

### Preparation of a library of NRTs and their evaluation in SBS polymerase and NRT conditions compatible with ion sensing.

*Preparation of Full Sets of NRTs Sufficient for All Studies in this Application:* Established methods are used to synthesize the NRTs for ion-sensing SBS evaluation (Ju et al. 2003; Ju et al. 2006; Wu et al. 2007; Guo et al. 2008).

*Characterization of Utility of NRTs for Ion Sensing:* The ion dependence for 9°N, Therminator II and Therminator III polymerases (all available from New England Biolabs, Ipswich, MA) that support incorporation of the NRTs are determined, initially using dideoxynucleotide triphosphates (ddNTPs) for single base extension reactions. Tests are performed in solution using synthetic template/primer systems, and cleaned-up extension products subjected to MALDI-TOF mass spectroscopy (MS) to quantify product yield. A series of monovalent and divalent cation, and monovalent anion concentrations, are tested. Once the basic parameters are established with dNTPs and ddNTPs, similar assays are performed using 3'-O-(2-nitrobenzyl), 3'-O-azidomethyl, and 3'-O-allyl nucleotides, utilizing enzymes that are best able to incorporate each of these modified nucleotides. Relevant time points are used to assess the salt dependence. While the salt-independent photo-cleavage of the 2-nitrobenzyl group may have advantages for the Ion Torrent-type system, automating chemical cleavage with azidomethyl or allyl derivatives is also possible.

To test polymerase specificity in the low salt buffer systems, all four ddNTPs or ddNTP analogues are combined in the reactions. In a synthetic template-primer system it is already known which of the 4 bases should be added next, and these can each be distinguished as well-separated peaks in the mass spectra. By including two or more of the same base in a row, these spectra are examined to confirm that reactions are terminated completely after the first base. Next, the buffer system used is tested with each of the preferred polymerase/nucleotide reversible terminator combinations. Reduction of the salt concentration to low enough amounts to permit subsequent ion sensing is also tested.

**NRTs tested in ion sensing platform.** When enzyme/NRT/low ion buffer systems are established, short runs of 2 or 3 base extensions are conducted on an H⁺ sensitive ion sensing system, such as the Ion Torrent, Inc. platform, as outlined in **Fig. 3****.** There is great flexibility in the number of samples that can be processed. Initially just a few different synthetic templates are employed. A range of the best buffer/salt conditions are used to maximize yields for ample detection by the ion sensor. Longer runs requiring larger amounts of NRTs are carried out under conditions giving the best results for the short runs. Templates can be attached to beads or directly to wells, and appropriate adapters are ligated if necessary to permit this. Artificial templates can be designed to test for specificity, dephasing (incomplete reactions or read-ahead), and ability to deal with long homopolymer sequences.

**Ion Sensor SBS with NRTs.** After confirmation that the ion sensing system handles a set of NRTs with good efficiency, a biological sample (a known viral or a bacterial genome) is sequenced using the combined SBS-ion sensing approach. Sequences are assembled and searched for the presence of polymorphisms or sequence errors. For example, pathogenic and non-pathogenic *Legionella* species can be used and a comparative analysis performed, with gene annotation as necessary.

The accuracy for homopolymer runs of more than a few bases is near perfect with the NRTs, but much lower with standard nucleotides. The need for cycles of incorporation, detection and cleavage adds additional time, but with automation and maximized efficiencies of both incorporation and deprotection, this does not outweigh the gain in accuracy. A ddNTP synchronization step can be included optionally in each or every other cycle. A sequence is assembled de *novo* for a low-repeat bacterial sequence. With appropriate long-range mate-pair library preparation methods, de *novo* and re-sequencing of eukaryotic genomes is also possible. Both long and short sequence reads are usable and the method can be employed for conducting comparative sequence analysis, genome assembly, annotation, and pathway analysis for prokaryotic and eukaryotic species.

### References:

Anderson, E. P. et al. (2008) A system for multiplexed direct electrical detection of DNA synthesis. Sens Actuators B Chem 129:78-86.
Bentley, D.R. et al. (2008) Accurate whole human genome sequencing using reversible terminator chemistry. Nature 456:53-59.
Bowers, J. et al. (2009) Virtual terminator nucleotides for next-generation DNA sequencing. Nature Methods, 6:593-595.
Branton, D. et al. (2008) The potential and challenges of nanopore sequencing. Nat Biotechnol 26:1146-1153.
Edwards, J.R. et al. (2001) DNA sequencing using biotinylated dideoxynucleotides and mass spectrometry. Nucleic Acids Res, 29:E104.
Eid, J. et al. (2008) Real-time DNA sequencing from single polymerase molecule. Science 323:133-138.
Fuller, C.W. et al. (2009) The challenges of sequencing by synthesis. Nat Biotechnol 27:1013-1023.
Gerstein, A.S., ed. Molecular Biology Problem Solver: A Laboratory Guide, Ch. 10, "Nucleotides, Oligonucleotides, and Polynucleotides" (2001).
Guo, J. et al. (2008) Four-color DNA sequencing with 3'-O-modified nucleotide reversible terminators and chemically cleavable fluorescent dideoxynucleotides. Proc Natl Acad Sci USA 105:9145-9150.
Haghighi, F. et al. (2008) Genetic architecture of the human tryptophan hydroxylase 2 gene: existence of neural isoforms and relevance for major depression. Mol Psychiatry. 13:813-820.
Harris, T.D. et al. (2008) Single-molecule DNA sequencing of a viral genome. Science, 320:106-109.
Hawkins, R.D. et al. (2010) Next-generation genomics: an integrative approach. Nat. Rev. Genet. 11:476-486.
Ju, J. et al. (1995) Energy transfer fluorescent dye-labeled primers for DNA sequencing and analysis. Proc Natl Acad Sci USA 92:4347-4351.
Ju, J. et al. (2003) Massive parallel method for decoding DNA and RNA. U.S. Patent 6,664,079.
Ju, J. et al. (2006) Four-color DNA sequencing by synthesis using cleavable fluorescent nucleotide reversible terminators. Proc Natl Acad Sci USA 103:19635-19640.
Landgraf, P. et al. (2007) A mammalian microRNA expression atlas based on small library RNA sequencing. Cell 129:1401-1414.
Li, Z. et al. (2003) A Photocleavable Fluorescent Nucleotide for DNA Sequencing and Analysis. Proc Natl Acad Sci USA 100:414-419.
Marti, A.A. et al. (2007) Design and characterization of two-dye and three-dye binary fluorescent probes for mRNA detection. Tetrahedron 63:3591-3600.
McKernan, K.J. et al. (2009) Sequence and structural variation in a human genome uncovered by short-read, massively parallel ligation sequencing using two base encoding. Genome Research 19:1527-1541.
Morozova, O. et al. (2009) Applications of new sequencing technologies for transcriptome analysis. Annu Rev Genomics Hum Genet 10:135-51.
Ng, S.B. et al. (2010) Massively parallel sequencing and rare disease. Hum Mol Genet 19:R19-R24.
Park, P.J. (2009) ChIP-seq: advantages and challenges of a maturing technology. Nat Rev Genet 10:669-680.
Ronaghi, M et al. (1998) A sequencing method based on real-time pyrophosphate. Science, 281:364-365.
Ronaghi, M. (2001) Pyrosequencing sheds light on DNA sequencing. Genome Res., 11:3-11.
Rothberg, J.M. et al. (2011) An integrated semiconductor device enabling non-optical genome sequencing. Nature 475:348-352.
Ruparel, H. et al. (2004) Digital detection of genetic mutations using SPC-sequencing. Genome Res. 14:296-300.
Ruparel, H. et al. (2005) Design and Synthesis of a 3'-O-allyl Photocleavable Fluorescent Nucleotide as a Reversible Terminator for DNA Sequencing By Synthesis. Proc Natl Acad Sci USA 102:5932-5937.
Sanger, F. et al. (1977) DNA sequencing with chain-terminating inhibitors. Proc Natl Acad Sci USA 74:5463-5467.
Seo, T.S et al. (2005) Four-Color DNA Sequencing by Synthesis on Chip Using Photocleavable Fluorescent Nucleotide Analogues. Proc Natl Acad Sci USA 102:5926-5931.
Shen, Y. et al. (2010) A SNP discovery method to assess variant allele probability from next-generation resequencing data. Genome Res 20:273-280.
Smith, L.M. et al. (1986) Fluorescence detection in automated DNA sequencing analysis. Nature 321:674-679.
Strug, L.J. et al. (2009) Centrotemporal sharp wave EEG trait in rolandic epilepsy maps to Elongator Protein Complex 4 (ELP4). Eur J Human Genet 17:1171-1181.
Wheeler, D.A. et al. (2008) The complete genome of an individual by massively parallel DNA sequencing. Nature 452:872-877.
Wu, J. et al. (2007) 3'-O-modified Nucleotides as Reversible Terminators for Pyrosequencing. Proc Natl Acad Sci USA 104:16462-16467.

## Claims

1. A method for determining the sequence of consecutive nucleotide residues in a single-stranded DNA in a solution comprising:
(a) contacting the single-stranded DNA, having a primer hybridized to a portion thereof, with a DNA polymerase and a deoxyribonucleotide triphosphate (dNTP) analogue base under conditions permitting the DNA polymerase to catalyze incorporation of the dNTP analogue into the primer if it is complementary to the nucleotide residue of the single-stranded DNA which is immediately 5' to a nucleotide residue of the single-stranded DNA hybridized to the 3' terminal nucleotide residue of the primer, so as to form a DNA extension product, wherein (1) the dNTP analogue has the structure: 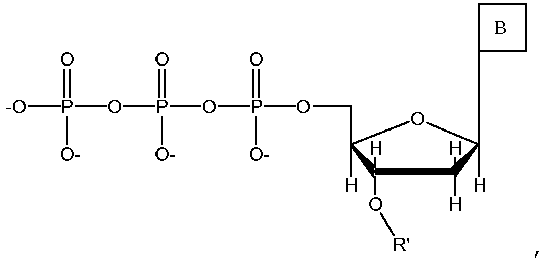 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the primer to form a DNA extension product, and if so, determining from the identity of the incorporated dNTP analogue the identity of the nucleotide residue in the single-stranded DNA complementary thereto, thereby determining the identity of the nucleotide residue in the single-stranded DNA, and wherein no change in hydrogen ion concentration indicates that the dNTP analogue has not been incorporated into the primer in step (a) ;
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the primer to form a DNA extension product, and determining from the identity of the incorporated dNTP analogue the identity of the nucleotide residue in the single-stranded DNA complementary thereto, thereby determining the identity of the nucleotide residue in the single-stranded DNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded DNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded DNA which is immediately 5' to a nucleotide residue of the single-stranded DNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
thereby determining the identity of each of the consecutive nucleotide residues of the single-stranded DNA so as to thereby determine the sequence of the consecutive nucleotide residues of the DNA.

2. A method for determining the sequence of consecutive nucleotide residues in a single-stranded RNA in a solution comprising:
(a) contacting the single-stranded RNA, having an RNA primer hybridized to a portion thereof, with a RNA polymerase and a ribonucleotide triphosphate (rNTP) analogue under conditions permitting the RNA polymerase to catalyze incorporation of the rNTP analogue into the RNA primer if it is complementary to the nucleotide residue of the single-stranded RNA which is immediately 5' to a nucleotide residue of the single-stranded RNA hybridized to the 3' terminal nucleotide residue of the RNA primer, so as to form an RNA extension product, wherein (1) the rNTP analogue has the structure: 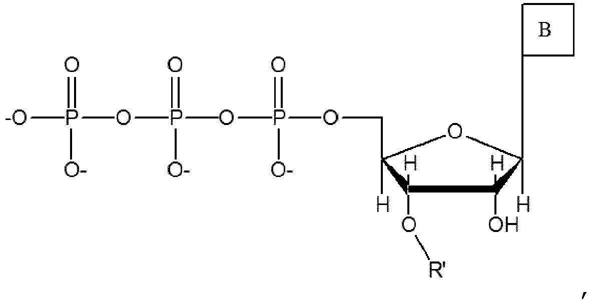 wherein B is a base and is adenine, guanine, cytosine, or uracil, and (2) R' is a hydrocarbyl, or a substituted hydrocarbyl, having a mass of less than 300 daltons and which is photochemically cleavable;
(b) determining whether incorporation of the rNTP analogue has occurred in step (a) by detecting an increase in hydrogen ion concentration of the solution, wherein an increase in hydrogen ion concentration indicates that the rNTP analogue has been incorporated into the RNA primer to form an RNA extension product, and if so, determining from the identity of the incorporated rNTP analogue the identity of the nucleotide residue in the single-stranded RNA complementary thereto, thereby determining the identity of the nucleotide residue in the single-stranded RNA, and wherein no change in hydrogen ion concentration indicates that the rNTP analogue has not been incorporated into the RNA primer in step (a);
(c) if no change in hydrogen ion concentration has been detected in step (b), iteratively performing steps (a) and (b), wherein in each iteration of step (a) for a given nucleotide residue, the identity of which is being determined, the rNTP analogue comprises a base which is a different type of base from the type of base of the rNTP analogues in every preceding iteration of step (a) for that nucleotide residue, until an rNTP analogue is incorporated into the RNA primer to form an RNA extension product, and determining from the identity of the incorporated rNTP analogue the identity of the nucleotide residue in the single-stranded RNA complementary thereto, thereby determining the identity of the nucleotide residue in the single-stranded RNA;
(d) if an increase in hydrogen ion concentration has been detected and an rNTP analogue is incorporated, subsequently treating the incorporated rNTP analogue with light to photochemically cleave the R' group so as to replace the R' group thereof with an H atom thereby providing a 3' OH group at the 3' terminal of the RNA extension product; and
(e) iteratively performing steps (a) to (d), as necessary, for each nucleotide residue of the consecutive nucleotide residues of the single-stranded RNA to be sequenced, except that in each repeat of step (a) the rNTP analogue is (i) incorporated into the RNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the RNA extension product resulting from a preceding iteration of step (a) or step (c), so as to form a subsequent RNA extension product, with the proviso that for the last nucleotide residue to be sequenced step (d) is optional,
thereby determining the identity of each of the consecutive nucleotide residues of the single-stranded RNA so as to thereby determine the sequence of the consecutive nucleotide residues of the RNA.

3. A method for determining the sequence of consecutive nucleotide residues in a single-stranded RNA in a solution comprising:
(a) contacting the single-stranded RNA, having a DNA primer hybridized to a portion thereof, with a reverse transcriptase and a deoxyribonucleotide triphosphate (dNTP) analogue under conditions permitting the reverse transcriptase to catalyze incorporation of the dNTP analogue into the primer if it is complementary to the nucleotide residue of the single-stranded RNA which is immediately 5' to a nucleotide residue of the single-stranded RNA hybridized to the 3' terminal nucleotide residue of the DNA primer, so as to form a DNA extension product, wherein (1) the dNTP analogue has the structure: 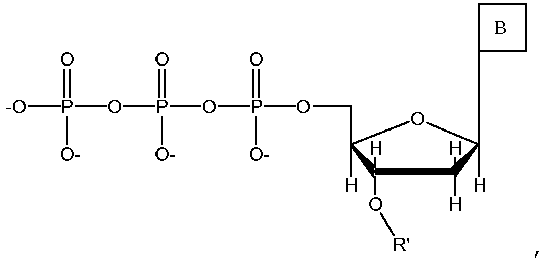 wherein B is a base and is adenine, guanine, cytosine, or thymine, and (2) R' is a hydrocarbyl, or a substituted hydrocarbyl, having a mass of less than 300 daltons and which is photochemically cleavable;
(b) determining whether incorporation of the dNTP analogue has occurred in step (a) by detecting an increase in hydrogen ion concentration of the solution, wherein an increase in hydrogen ion concentration indicates that the dNTP analogue has been incorporated into the DNA primer to form a DNA extension product, and if so, determining from the identity of the incorporated dNTP analogue the identity of the nucleotide residue in the single-stranded RNA complementary thereto, thereby determining the identity of the nucleotide residue in the single-stranded RNA, and wherein no change in hydrogen ion concentration indicates that the dNTP analogue has not been incorporated into the DNA primer in step (a);
(c) if no change in hydrogen ion concentration has been detected in step (b), iteratively performing steps (a) and (b), wherein in each iteration of step (a) for a given nucleotide residue, the identity of which is being determined, the dNTP analogue comprises a base which is a different type of base from the type of base of the dNTP analogues in every preceding iteration of step (a) for that nucleotide residue, until a dNTP analogue is incorporated into the DNA primer to form a DNA extension product, and determining from the identity of the incorporated dNTP analogue the identity of the nucleotide residue in the single-stranded RNA complementary thereto, thereby determining the identity of the nucleotide residue in the single-stranded RNA;
(d) if an increase in hydrogen ion concentration has been detected and a dNTP analogue is incorporated, subsequently treating the incorporated dNTP analogue with light to photochemically cleave the R' group so as to replace the R' group thereof with an H atom thereby providing a 3' OH group at the 3' terminal of the DNA extension product; and
(e) iteratively performing steps (a) to (d), as necessary, for each nucleotide residue of the consecutive nucleotide residues of the single-stranded RNA to be sequenced, except that in each repeat of step (a) the dNTP analogue is (i) incorporated into the DNA extension product resulting from a preceding iteration of step (a) or step (c), and (ii) complementary to a nucleotide residue of the single-stranded RNA which is immediately 5' to a nucleotide residue of the single-stranded RNA hybridized to the 3' terminal nucleotide residue of the DNA extension product resulting from a preceding iteration of step (a) or step (c), so as to form a subsequent DNA extension product, with the proviso that for the last nucleotide residue to be sequenced step (d) is optional,
thereby determining the identity of each of the consecutive nucleotide residues of the single-stranded RNA so as to thereby determine the sequence of the consecutive nucleotide residues of the RNA.

4. The method of any one of claims 1-3, wherein UV light is used to treat the R' group of a dNTP analogue or rNTP analogue incorporated into a primer or DNA or RNA extension product so as to photochemically cleave the moiety attached to the 3'-O so as to replace the 3'-O-R' with a 3'-OH.

5. The method of any one of claims 1-4, wherein R' is a substituted hydrocarbyl, and is a nitrobenzyl.

6. The method of any one of claims 1-5, wherein R' is a 2-nitrobenzyl, and wherein UV light is used to treat the R' group of a dNTP analogue or rNTP analogue incorporated into a primer or DNA or RNA extension product so as to photochemically cleave the moiety attached to the 3'-O so as to replace the 3'-O-R' with a 3'-OH, and wherein preferably the light is ∼355nm irradiation.

7. The method of any one of claims 1-6, wherein the single-stranded DNA or RNA is in a solution in a reaction chamber disposed on a sensor which is (i) formed in a semiconductor substrate and (ii) comprises a field-effect transistor or chemical field-effect transistor configured to provide at least one output signal in response to an increase in hydrogen ion concentration of the solution resulting from the formation of a phosphodiester bond between a dNTP or dNTP analogue or rNTP or rNTP analogue and a primer or a DNA or RNA extension product.

8. The method of claim 7, wherein the reaction chamber is one of a plurality of reaction chambers disposed on a sensor array formed in a semiconductor substrate and comprised of a plurality of sensors, each reaction chamber being disposed on at least one sensor and each sensor of the array comprising a field-effect transistor, or a chemical field-effect transistor, configured to provide at least one output signal in response to an increase in hydrogen ion concentration of the solution resulting from the formation of a phosphodiester bond between a dNTP, dNTP analogue, rNTP, or rNTP analogue and a primer or a DNA or RNA extension product.

9. The method of claim 8, wherein said sensors of said array each occupy an area of 100 µm or less and have a pitch of 10 µm or less and wherein each of said reaction chambers has a volume in the range of from 1 µm³ to 1500 µm³; wherein each of said reaction chambers contains at least 10⁵ copies of the single-stranded DNA or RNA in the solution; or wherein said plurality of said reaction chambers and said plurality of said sensors are each greater in number than 256,000.

10. The method of any one of claims 1-9, wherein single-stranded DNA(s) or RNA(s) in the solution are attached to a solid substrate; wherein a primer in the solution is attached to a solid substrate; wherein the single-stranded DNA or RNA or primer is attached to a solid substrate via 1,3-dipolar azide-alkyne cycloaddition chemistry; wherein the single-stranded DNA or RNA or primer is attached to a solid substrate via a polyethylene glycol molecule; wherein the single-stranded DNA or RNA or primer is attached to a solid substrate via a polyethylene glycol molecule and is azide-functionalized; wherein the single-stranded DNA or RNA or primer is attached to a solid substrate via an azido linkage, an alkynyl linkage, or biotin-streptavidin interaction; wherein the single-stranded DNA or RNA or primer is alkyne-labeled; wherein the single-stranded DNA or RNA or primer is attached to a solid substrate which is in the form of a chip, a bead, a well, a capillary tube, a slide, a wafer, a filter, a fiber, a porous media, a matrix, a porous nanotube, or a column; wherein the single-stranded DNA or RNA or primer is attached to a solid substrate which is a metal, gold, silver, quartz, silica, a plastic, polypropylene, a glass, nylon, or diamond; wherein the single-stranded DNA or RNA or primer is attached to a solid substrate which is a porous non-metal substance to which is attached or impregnated a metal or combination of metals; wherein the single-stranded DNA or RNA or primer is attached to a solid substrate which is in turn attached to a second solid substrate; or wherein the single-stranded DNA or RNA or primer is attached to a solid substrate which is in turn attached to a second solid substrate which is a chip.

11. The method of any one of claims 1-10, wherein 1x10⁹ or fewer copies of the single-stranded DNA or RNA or primer are attached to a solid substrate; wherein 1x10⁸ or fewer copies of the single-stranded DNA or RNA or primer are attached to a solid substrate; wherein 2x10⁷ or fewer copies of the single-stranded DNA or RNA or primer are attached to a solid substrate; wherein 1x10⁷ or fewer copies of the single-stranded DNA or RNA or primer are attached to a solid substrate; wherein 1x10⁶ or fewer copies of the single-stranded DNA or RNA or primer are attached to a solid substrate; wherein 1x10⁴ or fewer copies of the single-stranded DNA or RNA or primer are attached to a solid substrate; or wherein 1,000 or fewer copies of the single-stranded DNA or RNA or primer are attached to a solid substrate.

12. The method of any one of claims 1-10, wherein 10,000 or more copies of the single-stranded DNA or RNA or primer are attached to a solid substrate; wherein 1x10⁷ or more copies of the DNA or RNA or primer are attached to a solid substrate; wherein 1x10⁸ or more copies of the single-stranded DNA or RNA or primer are attached to a solid substrate; wherein 1x10⁹ or more copies of the single-stranded DNA or RNA or primer are attached to a solid substrate; or wherein the single-stranded DNA or RNA or primer are separated in discrete compartments, wells, or depressions on a solid surface.

13. The method of any one of claims 1-12 performed in parallel on a plurality of single-stranded DNAs or RNAs; and wherein optionally the single-stranded DNAs or RNAs are templates having the same sequence.

14. The method of claim 13, further comprising after the residue of the nucleotide residue has been determined in step (b), or (c), as appropriate, permanently capping any unextended primers or unextended DNA or RNA extension products.

15. The method of any one of claims 13 or 14, wherein the single-stranded DNA or RNA is amplified from a sample of DNA or RNA prior to step (a); and wherein optionally the single-stranded DNA or RNA is amplified by polymerase chain reaction.

## Patentansprüche

1. Verfahren zur Bestimmung der Sequenz von aufeinanderfolgenden Nukleotidresten in einer einzelsträngigen DNA in einer Lösung umfassend:
(a) Inkontaktbringen der einzelsträngigen DNA mit einem an einem Teil davon hybridisierenden Primer mit einer DNA-Polymerase und einer Desoxyribonukleotidtriphosphat (dNTP)-Analogbase unter Bedingungen, die es der DNA-Polymerase ermöglichen, den Einbau des dNTP-Analogs in den Primer zu katalysieren, wenn es zu dem Nukleotidrest der einzelsträngigen DNA komplementär ist, der unmittelbar 5' zu einem Nukleotidrest der einzelsträngigen DNA ist, der mit dem 3'-terminalen Nukleotidrest des Primers hybridisiert, um ein DNA-Verlängerungsprodukt zu bilden, wobei (1) das dNTP-Analog die folgende Struktur aufweist: 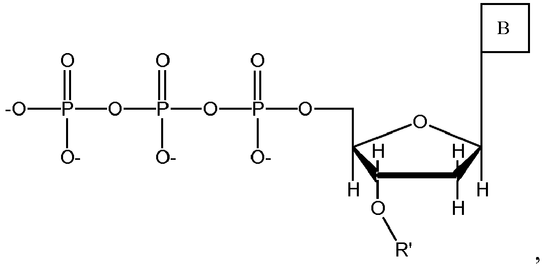 wobei B eine Base ist und Adenin, Guanin, Cytosin, oder Thymin ist, und (2) R' ein Hydrocarbyl, oder ein substituiertes Hydrocarbyl mit einer Masse von weniger als 300 Daltons ist und welches photochemisch spaltbar ist;
(b) Bestimmen ob der Einbau des dNTP-Analogs in Schritt (a) erfolgt ist in dem bestimmt wird, ob ein Anstieg der Wasserstoffionenkonzentration der Lösung aufgetreten ist, wobei ein Anstieg der Wasserstoffionenkonzentration aufweist, dass das dNTP-Analog in den Primer eingebaut ist um ein DNA-Verlängerungsprodukt zu bilden, und falls ja, Bestimmen des Nukleotidrests in einem einzelsträngigen DNA-Komplementär aus der Identität des eingebauten dNTP-Analogs, dabei bestimmend die Identität des Nukleotidrests in der einzelsträngigen DNA, und wobei kein Unterschied in der Wasserstoffionenkonzentration aufweist, dass das dNTP-Analog nicht in dem Primer in Schritt (a) eingebaut wurde;
(c) Falls kein Unterschied in der Wasserstoffionenkonzentration in Schritt (b) bestimmt wurde, iterative Durchführungsschritte (a) und (b), wobei in jedem Iterationsschritt (a) für einen bestimmten Nukleotidrest, in welchem die Identität bestimmt wird, der dNTP-Analog eine Base umfasst, welche ein unterschiedlicher Typ zu der Base von dem Typ von der Base des dNTP-Analogs in jeder vorangegangenen Iteration des Schrittes (a) für diesen Nukleotidrest, bis ein dNTP-Analog in den Primer eingebaut ist um ein DNA-Verlängerungsprodukt zu bilden und Bestimmen des Nukleotidrests in dem einzelsträngigen DNA-Komplementär aus der Identität des eingebauten dNTP-Analogs, dabei bestimmend die Identität des Nukleotidrests in der einzelsträngigen DNA;
(d) Falls eine Erhöhung der Wasserstoffionenkonzentration bestimmt wurde und ein dNTP-Analog eingebaut wurde, nachfolgend Behandeln des eingebauten dNTP-Analogs mit Licht um die R'-Gruppe photochemisch zu spalten, so dass die R'-Gruppe mit einem H-Atom ersetzt wird und eine 3'-OH-Gruppe an dem 3'-Terminus des DNA-Verlängerungsprodukts zur Verfügung gestellt wird; und
(e) iterative Durchführungsschritte (a) bis (d), nach Bedarf um jeden Nukleotidrest des aufeinanderfolgenden Nukleotidrests von einem einzelsträngigen DNA zu sequenzieren, außer dass in jeder Wiederholung des Schrittes (a) das dNTP-Analog ist (i) eingebaut in ein DNA-Verlängerungsprodukt, welches aus der vorangegangenen Iteration des Schrittes (a) oder Schrittes (c), und (ii) einen Nukleotidrest von einer einzelsträngigen DNA-Komplementär erfolgt welcher unmittelbar 5' zu einem Nukleotidrest der einzelsträngigen DNA hybridisiert zu einem 3'-terminalen Nukleotidrest von dem DNA-Verlängerungsprodukt welcher aus der vorangegangen Iteration des Schrittes (a) oder Schrittes (c) erfolgt, so dass ein nachfolgendes DNA-Verlängerungsprodukt gebildet wird, mit der Ausnahme, dass zu dem letzten Nukleotidrest welcher sequenziert wird Schritt (d) optional ist,
dabei bestimmend die Identität von jedem von den aufeinanderfolgenden Nukleotidresten in einer einzelsträngigen DNA so dass dabei die Sequenz des aufeinanderfolgenden Nukleotidrests von der DNA bestimmt wird.

2. Verfahren zur Bestimmung der Sequenz von aufeinanderfolgenden Nukleotidresten in einer einzelsträngigen RNA in einer Lösung umfassend:
(a) Inkontaktbringen der einzelsträngigen RNA mit einem an einem Teil davon hybridisierenden RNA-Primer mit einer RNA-Polymerase und einem Ribonukleotidtriphosphat (rNTP)-Analog unter Bedingungen, die es der RNA-Polymerase ermöglichen, den Einbau des rNTP-Analogs in den Primer zu katalysieren, wenn es zu dem Nukleotidrest der einzelsträngigen RNA komplementär ist, der unmittelbar 5' zu einem Nukleotidrest der einzelsträngigen RNA ist, der mit dem 3'-terminalen Nukleotidrest des RNA-Primers hybridisiert, um ein RNA-Verlängerungsprodukt zu bilden, wobei (1) das rNTP-Analog die folgende Struktur aufweist: 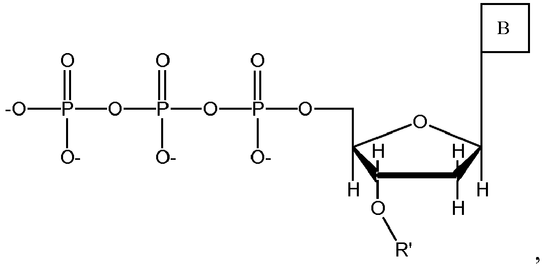 wobei B eine Base ist und Adenin, Guanin, Cytosin, oder Uracil ist, und (2) R' ein Hydrocarbyl, oder ein substituiertes Hydrocarbyl mit einer Masse von weniger als 300 Daltons ist und welches photochemisch spaltbar ist;
(b) Bestimmen ob der Einbau des rNTP-Analogs in Schritt (a) erfolgt ist in dem bestimmt wird, ob ein Anstieg der Wasserstoffionenkonzentration der Lösung aufgetreten ist, wobei ein Anstieg der Wasserstoffionenkonzentration aufweist, dass das rNTP-Analog in dem RNA-Primer eingebaut ist um ein RNA-Verlängerungsprodukt zu bilden, und falls ja, Bestimmen der Identität des Nukleotidrests in dem einzelsträngigen RNA-Komplementär aus der Identität des eingebauten rNTP-Analogs, dabei bestimmend die Identität des Nukleotidrests in einer einzelsträngigen RNA, und wobei kein Unterschied in Wasserstoffionenkonzentration aufweist, dass das rNTP-Analog nicht in den RNA-Primer in Schritt (a) eingebaut wurde;
(c) Falls kein Unterschied in der Wasserstoffionenkonzentration in Schritt (b) bestimmt wurde, iterative Durchführungsschritte (a) und (b), wobei in jedem Iterationsschritt (a) zu einem bestimmten Nukleotidrest, in welchem die Identität bestimmt wird, der rNTP-Analog eine Base umfasst, welche ein unterschiedlicher Typ zu der Base von dem Typ von der Base des rNTP-Analogs in jeder vorangegangenen Iteration des Schrittes (a) für diesen Nukleotidrest ist, bis ein rNTP-Analog in den RNA-Primer eingebaut ist um ein RNA-Verlängerungsprodukt zu bilden und Bestimmen der Identität des eingebauten rNTP-Analogs die Identität des Nukleotidrests in dem einzelsträngigen RNA Komplementär, dabei bestimmend die Identität des Nukleotidrests in der einzelsträngigen RNA;
(d) Falls eine Erhöhung der Wasserstoffionenkonzentration bestimmt wurde und ein rNTP-Analog eingebaut wurde, nachfolgend Behandeln des eingebauten rNTP-Analogs mit Licht um die R'-Gruppe photochemisch zu spalten, so dass die R'-Gruppe davon mit einem H-Atom ersetzt wird und dabei eine 3'-OH-Gruppe an dem 3'-Terminus des RNA-Verlängerungsprodukts zur Verfügung zu stellen; und
(e) iterative Durchführungsschritte (a) bis (d), nach Bedarf, um jeden Nukleotidrest des aufeinanderfolgenden Nukleotidrests von einem einzelsträngigen RNA zu sequenzieren, außer dass in jeder Wiederholung des Schrittes (a) ein rNTP Analog ist (i) eingebaut in ein RNA-Verlängerungsprodukt, welches aus der vorangegangenen Iteration des Schrittes (a) oder Schrittes (c), und (ii) einen Komplementär von einem Nukleotidrest von einer einzelsträngigen RNA erfolgt welcher unmittelbar 5' zu einem Nukleotidrest zu einer einzelsträngigen RNA der hybridisiert zu einem 3'terminalen Nukleotidrest von dem RNA-Verlängerungsprodukt welcher aus der vorangegangen Iteration des Schrittes (a) oder Schrittes (c) erfolgt, so dass ein nachfolgendes RNA-Verlängerungsprodukt gebildet wird, mit der Ausnahme, dass zu dem letzten Nukleotidrest welcher sequenziert wird, Schritt (d) optional ist,
dabei bestimmend die Identität von jedem von den aufeinanderfolgenden Nukleotidresten in der einzelsträngigen RNA so dass die Sequenz des aufeinanderfolgenden Nukleotidrests von der RNA bestimmt wird.

3. Verfahren zur Bestimmung der Sequenz von aufeinanderfolgenden Nukleotidresten in einer einzelsträngigen RNA in einer Lösung umfassend:
(a) Inkontaktbringen der einzelsträngigen RNA mit einem an einem Teil davon hybridisierenden DNA-Primer mit einer Reversetranskriptase und einem Deoxyribonukleotidtriphosphat (dNTP)-Analog unter Bedingungen, die es der Reversetranskriptase ermöglichen, den Einbau des dNTP Analogs in den Primer zu katalysieren, wenn es zu dem Nukleotidrest der einzelsträngigen RNA komplementär ist, der unmittelbar 5' zu einem Nukleotidrest der einzelsträngigen RNA ist, der mit dem 3'-terminalen Nukleotidrest des DNA-Primers hybridisiert, um ein DNA-Verlängerungsprodukt zu bilden, wobei (1) das dNTP-Analog die folgende Struktur aufweist: 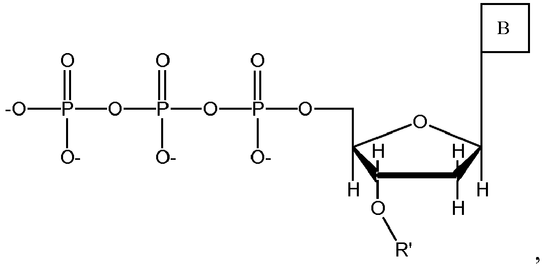 wobei B eine Base ist und Adenin, Guanin, Zytosin, oder Thymin ist, und (2) R' ein Hydrocarbyl, oder ein substituiertes Hydrocarbyl mit einer Masse von weniger als 300 Daltons ist und welches photochemisch spaltbar ist;
(b) Bestimmen ob der Einbau des dNTP-Analogs in Schritt (a) erfolgt ist in dem bestimmt wird, ob ein Anstieg der Wasserstoffionenkonzentration der Lösung aufgetreten ist, wobei ein Anstieg der Wasserstoffionenkonzentration aufweist, dass das dNTP-Analog in dem DNA-Primer eingebaut ist um ein DNA-Verlängerungsprodukt zu bilden, und falls ja, Bestimmen der Identität des Nukleotidrests in dem einzelsträngigen RNA-Komplementär aus der Identität des eingebauten dNTP-Analogs, dabei bestimmend die Identität des Nukleotidrests in der einzelsträngigen RNA, und wobei kein Unterschied der Wasserstoffionenkonzentration aufweist, dass das dNTP- Analog nicht in dem DNA-Primer in Schritt (a) eingebaut wurde;
(c) Falls kein Unterschied der Wasserstoffionenkonzentration in Schritt (b) bestimmt wurde, iterative Durchführungsschritte (a) und (b), wobei in jedem Iterationsschritt (a) zu einem bestimmten Nukleotidrest, in welchem die Identität bestimmt wird, der dNTP-Analog eine Base umfasst, welche ein unterschiedlicher Typ zu der Base von dem Typ von der Base der dNTP-Analoge ist in jeder vorangegangenen Iteration des Schrittes (a) für diesen Nukleotidrest, bis ein dNTP-Analog in einem DNA-Primer eingebaut ist um ein DNA-Verlängerungsprodukt zu bilden und Bestimmen der Identität des Nukleotidrests in dem einzelsträngigen RNA-Komplementär aus der Identität des eingebauten dNTP-Analogs, dabei bestimmend die Identität des Nukleotidrests in der einzelsträngigen RNA;
(d) Falls eine Erhöhung der Wasserstoffionenkonzentration bestimmt wurde und ein dNTP-Analog eingebaut wurde, nachfolgend Behandeln des eingebauten dNTP-Analogs mit Licht um die R'-Gruppe photochemisch zu spalten, so dass die R'-Gruppe davon mit einem H-Atom besetzt wird und dabei eine 3'-OH-Gruppe an dem 3'-Terminus des DNA-Verlängerungsprodukts zur Verfügung zu stellen; und
(e) iterative Durchführungsschritte (a) bis (d), nach Bedarf, um jeden Nukleotidrest des aufeinanderfolgenden Nukleotidrests von einem einzelsträngigen RNA zu sequenzieren, außer dass in jeder Wiederholung des Schrittes (a) ein dNTP Analog ist (i) eingebaut in ein DNA-Verlängerungsprodukt, welches aus der vorangegangenen Iteration des Schrittes (a) oder Schrittes (c), und (ii) komplementär zu einem Nukleotidrest von der einzelsträngigen RNA erfolgt, welche unmittelbar 5' zu einem Nukleotidrest zu einer einzelsträngigen RNA die hybridisiert zu einem 3'-terminalen Nukleotidrest von dem DNA-Verlängerungsprodukt welcher aus der vorangegangen Iteration des Schrittes (a) oder Schrittes (c) erfolgt, so dass ein nachfolgendes DNA-Verlängerungsprodukt gebildet wird, mit der Ausnahme, dass zu dem letzten Nukleotidrest, welcher sequenziert wird, Schritt (d) optional ist,
dabei bestimmend die Identität von jedem von den aufeinanderfolgenden Nukleotidresten in einer einzelsträngigen RNA, so dass die Sequenz der aufeinanderfolgenden Nukleotidreste von RNA bestimmt wird.

4. Verfahren gemäß einem der Ansprüche 1-3, wobei UV-Licht verwendet wird um die R' Gruppe von einem dNTP-Analog oder rNTP-Anlog eingebaut in einen Primer oder DNA- oder RNA- Verlängerungsprodukt zu behandeln, so dass der Rest verbunden zu der 3'-O photochemisch gespalten wird, so dass das 3'-O-R' mit einem 3'-OH ersetzt wird.

5. Verfahren gemäß einem der Ansprüche 1-4, wobei R' ein substitutiertes Hydrocarbyl ist und ein Nitrobenzyl ist.

6. Verfahren von einem der Ansprüche 1-5, wobei R' ein 2-Nitrobenzyl ist und wobei UV-Licht verwendet wird um die R'-Gruppe von einem dNTP-Analog oder rNTP-Analog eingebaut in einen Primer oder DNA- oder RNA- Verlängerungsprodukt zu behandeln, so dass der Rest verbunden mit 3'-O photochemisch gespalten wird, so dass 3'-O-R' mit einem 3'-OH ersetzt wird und wobei bevorzugt das Licht ∼355nm Strahlung ist.

7. Verfahren gemäß einem der Ansprüche 1-6, wobei die einzelsträngige DNA oder RNA in einer Lösung in einer Reaktionskammer disponiert auf einem Sensor ist, welcher ist (i) geformt in einem Halbleitersubstrat und (ii) umfasst einen Feldeffekttransistor oder einen chemischen Feldeffekttransistor konfiguriert um mindestens ein Outputsignal zur Antwort zur Erhöhung der Wasserstoffionenkonzentration der Lösung ergeben aus der Bildung von einer Phosphordiesterbindung zwischen einem dNTP oder dNTP-Analog oder rNTP oder rNTP-Analog und einem Primer oder einem DNA- oder RNA-Verlängerungsprodukt.

8. Verfahren gemäß Anspruch 7, wobei die Reaktionskammer eine Mehrzahl von Reaktionskammern ist angeordnet auf einer Sensorreihe geformt in einem Halbleitersubstrat und umfasst von einer Mehrzahl von Sensoren, jede Reaktionskammer angeordnet bei mindestens einem Sensor und jeder Sensor der Reihe umfassend einen Feldeffekttransistor oder einen chemischen Feldeffekttransistor konfiguriert um mindestens ein Outputsignal zur Verfügung zu stellen zur Antwort zur Erhöhung der Wasserstoffionenkonzentration der Lösung ergeben aus der Bildung von einer Phosphordiesterbindung zwischen einem dNTP, dNTP-Analog, rNTP oder rNTP-Analog und einem Primer oder einem DNA- oder RNA-Verlängerungsprodukt.

9. Verfahren gemäß Anspruch 8, wobei die Sensoren von jeder Reihe eine Fläche von 100 µm oder weniger haben und einen Abstand von 10 µm oder weniger haben und wobei jede dieser Reaktionskammern ein Volumen im Bereich von 1 µm bis 1500 µm³ hat; wobei jede dieser Reaktionskammern mindestens 10⁵ Kopien von einer einzelsträngigen DNA oder RNA in der Lösung enthält; oder wobei die Mehrzahl von diesen Reaktionskammern und Mehrzahl von Sensoren jeder eine Zahl höher als 256.000 aufweist.

10. Verfahren gemäß einem der Ansprüche 1-9, wobei einzelsträngige DNA(s) oder RNA(s) in der Lösung zu einem festen Substrat verbunden sind; wobei ein Primer in der Lösung zu einem festen Substrat verbunden ist; wobei die einzelsträngige DNA oder RNA oder Primer zu einem festen Substrat via 1,3-dipolare Azid-Alkyn Cycloadditionschemie verbunden ist; wobei die einzelsträngige DNA oder RNA oder Primer zu einem festen Substrat via Polyethylenglykolmolekül verbunden ist; wobei die einzelsträngige DNA oder RNA oder Primer zu einem festen Substrat via Polyethylenglykolmolekül verbunden ist und Azid funktionalisiert ist; wobei die einzelsträngige DNA oder RNA oder Primer zu einem festen Substrat via einer Azidobindung, einer Alkynylbindung oder Biotin-streptavidininteraktion verbunden ist; wobei die einzelsträngige DNA oder RNA oder Primer Alkyn-markiert ist; wobei die einzelsträngige DNA oder RNA oder Primer zu einem festen Substrat verbunden ist, welches in einer Form von einem Chip ist, einer Perle, einem Schacht, einem Kapillarrohr, ein Gleiten, ein Objektträger, ein Wafer, ein Filter, eine Faser, ein pöroses Medium, eine Matrix, ein pöröses Nanoröhrchen oder eine Säule aufweist; wobei die einzelsträngigen DNA oder RNA oder Primer an ein festes Substrat gebunden ist, das ein Metall, Gold, Silber, Quarz, Siliziumoxid, Kunststoff, Polypropylen, Glas, Nylon oder Diamant ist; wobei die einzelsträngige DNA oder RNA oder Primer an ein festes Substrat gebunden ist, dass eine pörose nicht Metallsubstanz ist, an die ein imprägniertes Metall oder Kombination von Metallen gebunden ist; wobei die einzelsträngige DNA oder RNA oder Primer in ein festes Substrat gebunden ist der seinerseits an ein zweites festes Substrat gebunden ist oder wobei die einzelsträngige DNA oder RNA oder Primer an ein festes Substrat gebunden ist, das seinerseits an ein zweites festes Substrat gebunden ist, das ein Chip ist.

11. Verfahren gemäß einem der Ansprüche 1-10, wobei 1x10⁹ oder weniger Kopien des einzelsträngigen DNA oder RNA oder Primer zu einem festen Substrat verbunden sind; wobei 1x10⁸ oder weniger Kopien des einzelsträngigen DNA oder RNA oder Primer zu einem festen Substrat verbunden sind; wobei 2x10⁷ oder weniger Kopien der einzelsträngigen DNA oder RNA oder Primer zu einem festen Substrat verbunden sind, wobei 1x10⁷ oder weniger Kopien der einzelsträngigen DNA oder RNA oder Primer zu einem festen Substrat verbunden sind; wobei 1x10⁶ oder weniger Kopien der einzelsträngigen DNA oder RNA oder Primer zu einem festen Substrat verbunden sind; wobei 1x10⁴ oder weniger Kopien der einzelsträngigen DNA oder RNA oder Primer zu einem festen Substrat verbunden sind; oder wobei 1.000 oder weniger Kopien der einzelsträngigen DNA oder RNA oder Primer zu einem festen Substrat verbunden sind.

12. Verfahren gemäß einem der Ansprüche 1-10, wobei 10.000 oder mehr Kopien der einzelsträngigen DNA oder RNA oder Primer zu einem festen Substrat befestigt sind; wobei 1x10⁷ oder mehr Kopien von der DNA oder RNA oder Primer zu einem festen Substrat befestigt sind; wobei 1x10⁸ oder mehr Kopien der einzelsträngigen DNA oder RNA oder Primer zu einem festen Substrat befestigt sind; wobei 1x10⁹ oder mehr Kopien der einzelsträngigen DNA oder RNA oder Primer zu einem festen Substrat befestigt sind; oder wobei die einzelsträngige DNA oder RNA oder Primer in separate Kammern, Schächte, oder Absenkungen auf einer festen Oberfläche getrennt sind.

13. Verfahren gemäß einem der Ansprüche 1-12 durchgeführt parallel auf einer Vielzahl von einzelsträngigen DNAs oder RNAs; und wobei optional die einzelsträngigen DNAs oder RNAs Template sind, mit der gleichen Sequenz.

14. Verfahren gemäß Anspruch 13, ferner umfassend nachdem in Schritt (b), oder (c) der Rest des Nukleotidrests bestimmt wurde, wenn anwendbar, permanent Schließen nicht-verlängerter Primer oder nicht-verlängerte DNA oder RNA-Verlängerungsprodukte.

15. Verfahren gemäß einem der Ansprüche 13 oder 14, wobei die einzelsträngige DNA oder RNA aus einer Probe von DNA oder RNA vor Schritt (a) amplifiziert wird; und wobei optional der einzelsträngige DNA oder RNA mit einer Polymerasekettenreaktion amplifiziert wird.

## Revendications

1. Procédé de détermination de la séquence de résidus nucléotidiques consécutifs dans un ADN simple brin dans une solution comprenant :
(a) la mise en contact de l'ADN simple brin, ayant une amorce hybridée à une partie de ce dernier, avec une ADN polymérase et une base analogue de désoxyribonucléotide triphosphate (dNTP) dans des conditions permettant à l'ADN polymérase de catalyser l'incorporation de l'analogue de dNTP dans l'amorce s'il est complémentaire du résidu nucléotidique de l'ADN simple brin qui est immédiatement en 5' par rapport à un résidu nucléotidique de l'ADN simple brin hybridé au résidu nucléotidique terminal 3' de l'amorce, de manière à former un produit d'extension d'ADN, dans lequel (1) l'analogue de dNTP a la structure : 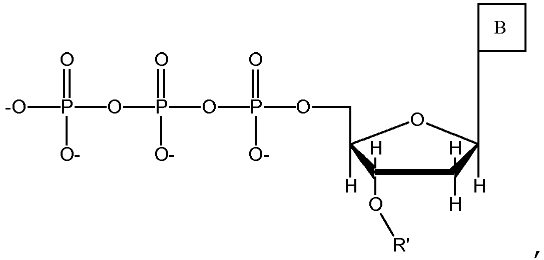 dans laquelle B est une base et est l'adénine, la guanine, la cytosine ou la thymine, et (2) R' est un hydrocarbyle, ou un hydrocarbyle substitué, ayant une masse inférieure à 300 daltons et qui peut être clivé par voie photochimique ;
(b) la détermination si l'incorporation de l'analogue de dNTP s'est produite à l'étape (a) par détection d'une augmentation de la concentration en ions hydrogène de la solution, dans laquelle une augmentation de la concentration en ions d'hydrogène indique que l'analogue de dNTP a été incorporé dans l'amorce pour former un produit d'extension d'ADN, et si tel est le cas, la détermination à partir de l'identité de l'analogue de dNTP incorporé de l'identité du résidu nucléotidique dans l'ADN simple brin qui lui est complémentaire, déterminant ainsi l'identité du résidu nucléotidique dans l'ADN simple brin, et dans laquelle l'absence de changement de la concentration en ions hydrogène indique que l'analogue de dNTP n'a pas été incorporé dans l'amorce à l'étape (a) ;
(c) si aucune modification de la concentration en ions hydrogène n'a été détectée à l'étape (b), la réalisation de manière itérative des étapes (a) et (b), dans lesquelles, à chaque itération de l'étape (a), pour un résidu nucléotidique donné, dont l'identité est à déterminer, l'analogue de dNTP comprend une base qui est un type de base différent du type de base des analogues de dNTP dans chaque itération précédente de l'étape (a) pour ce résidu nucléotidique, jusqu'à ce qu'un analogue de dNTP soit incorporé dans l'amorce pour former un produit d'extension d'ADN, et la détermination à partir de l'identité de l'analogue de dNTP incorporé de l'identité du résidu nucléotidique dans l'ADN simple brin qui lui est complémentaire, déterminant ainsi l'identité du résidu nucléotidique dans l'ADN simple brin ;
(d) si une augmentation de la concentration en ions hydrogène a été détectée et qu'un analogue de dNTP est incorporé, le traitement par la suite de l'analogue de dNTP incorporé avec de la lumière afin de cliver par voie photochimique le groupe R' de manière à remplacer son groupe R' par un atome de H, fournissant ainsi un groupe OH en 3' à l'extrémité terminale 3' du produit d'extension d'ADN ; et
(e) la réalisation de manière itérative des étapes (a) à (d), si nécessaire, pour chaque résidu nucléotidique des résidus nucléotidiques consécutifs de l'ADN simple brin à séquencer, sauf que dans chaque répétition de l'étape (a), l'analogue de dNTP est (i) incorporé dans le produit d'extension d'ADN résultant d'une itération précédente de l'étape (a) ou de l'étape (c), et (ii) complémentaire d'un résidu nucléotidique de l'ADN simple brin qui est immédiatement en 5' d'un résidu nucléotidique de l'ADN simple brin hybridé au résidu nucléotidique terminal 3' du produit d'extension d'ADN résultant d'une itération précédente de l'étape (a) ou de l'étape (c), de manière à former un produit d'extension d'ADN ultérieur, à condition que pour le dernier résidu nucléotidique à séquencer l'étape (d) soit facultative,
déterminant ainsi l'identité de chacun des résidus nucléotidiques consécutifs de l'ADN simple brin de manière à déterminer ainsi la séquence des résidus nucléotidiques consécutifs de l'ADN.

2. Procédé de détermination de la séquence de résidus nucléotidiques consécutifs dans un ARN simple brin dans une solution comprenant :
(a) la mise en contact de l'ARN simple brin, ayant une amorce d'ARN hybridée à une partie de ce dernier, avec une ARN polymérase et un analogue de ribonucléotide triphosphate (rNTP) dans des conditions permettant à l'ARN polymérase de catalyser l'incorporation de l'analogue de rNTP dans l'amorce d'ARN s'il est complémentaire du résidu nucléotidique de l'ARN simple brin qui est immédiatement en 5' par rapport à un résidu nucléotidique de l'ARN simple brin hybridé au résidu nucléotidique terminal 3' de l'amorce d'ARN, de manière à former un produit d'extension d'ARN, dans lequel (1) l'analogue de rNTP a la structure : 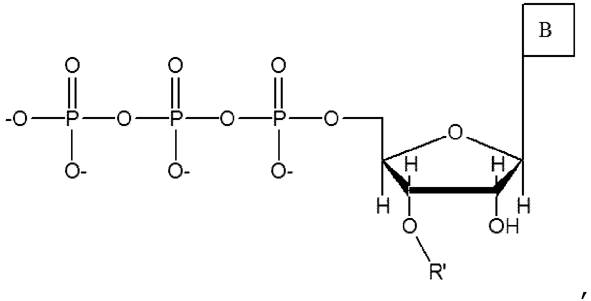 dans laquelle B est une base et est l'adénine, la guanine, la cytosine ou l'uracile, et (2) R' est un hydrocarbyle, ou un hydrocarbyle substitué, ayant une masse inférieure à 300 daltons et qui peut être clivé par voie photochimique ;
(b) la détermination si l'incorporation de l'analogue de rNTP s'est produite à l'étape (a) par détection d'une augmentation de la concentration en ions hydrogène de la solution, dans laquelle une augmentation de la concentration en ions d'hydrogène indique que l'analogue de rNTP a été incorporé dans l'amorce d'ARN pour former un produit d'extension d'ARN, et si tel est le cas, la détermination à partir de l'identité de l'analogue de rNTP incorporé de l'identité du résidu nucléotidique dans l'ARN simple brin qui lui est complémentaire, déterminant ainsi l'identité du résidu nucléotidique dans l'ARN simple brin, et dans laquelle l'absence de changement dans la concentration en ions hydrogène indique que l'analogue de rNTP n'a pas été incorporé dans l'amorce d'ARN à l'étape (a) ;
(c) si aucune modification de la concentration en ions hydrogène n'a été détectée à l'étape (b), la réalisation de manière itérative des étapes (a) et (b), dans lesquelles, à chaque itération de l'étape (a), pour un résidu nucléotidique donné, dont l'identité est à déterminer, l'analogue de rNTP comprend une base qui est un type de base différent du type de base des analogues de rNTP dans chaque itération précédente de l'étape (a) pour ce résidu nucléotidique, jusqu'à ce qu'un analogue de rNTP soit incorporé dans l'amorce d'ARN pour former un produit d'extension d'ARN, et la détermination à partir de l'identité de l'analogue rNTP incorporé de l'identité du résidu nucléotidique dans l'ARN simple brin qui lui est complémentaire, déterminant ainsi l'identité du résidu nucléotidique dans l'ARN simple brin ;
(d) si une augmentation de la concentration en ions hydrogène a été détectée et qu'un analogue de rNTP est incorporé, le traitement par la suite de l'analogue de rNTP incorporé avec de la lumière afin de cliver par voie photochimique le groupe R' de manière à remplacer son groupe R' par un atome de H, fournissant ainsi un groupe OH en 3' à l'extrémité terminale 3' du produit d'extension d'ARN ; et
(e) la réalisation de manière itérative des étapes (a) à (d), si nécessaire, pour chaque résidu nucléotidique des résidus nucléotidiques consécutifs de l'ARN simple brin à séquencer, sauf que dans chaque répétition de l'étape (a), l'analogue de rNTP est (i) incorporé dans le produit d'extension d'ARN résultant d'une itération précédente de l'étape (a) ou de l'étape (c), et (ii) complémentaire d'un résidu nucléotidique de l'ARN simple brin qui est immédiatement en 5' d'un résidu nucléotidique de l'ARN simple brin hybridé au résidu nucléotidique terminal 3' du produit d'extension d'ARN résultant d'une itération précédente de l'étape (a) ou de l'étape (c), de manière à former un produit d'extension d'ARN ultérieur, à condition que pour le dernier résidu nucléotidique à séquencer l'étape (d) soit facultative,
déterminant ainsi l'identité de chacun des résidus nucléotidiques consécutifs de l'ARN simple brin de manière à déterminer ainsi la séquence des résidus nucléotidiques consécutifs de l'ARN.

3. Procédé de détermination de la séquence de résidus nucléotidiques consécutifs dans un ARN simple brin dans une solution comprenant :
(a) la mise en contact de l'ARN simple brin, ayant une amorce d'ADN hybridée à une partie de ce dernier, avec une transcriptase inverse et un analogue de désoxyribonucléotide triphosphate (dNTP) dans des conditions permettant à la transcriptase inverse de catalyser l'incorporation de l'analogue de dNTP dans l'amorce, s'il est complémentaire du résidu nucléotidique de l'ARN simple brin qui est immédiatement en 5' par rapport à un résidu nucléotidique de l'ARN simple brin hybridé au résidu nucléotidique terminal 3' de l'amorce d'ADN, de manière à former un produit d'extension d'ADN, dans lequel (1) l'analogue de dNTP a la structure : 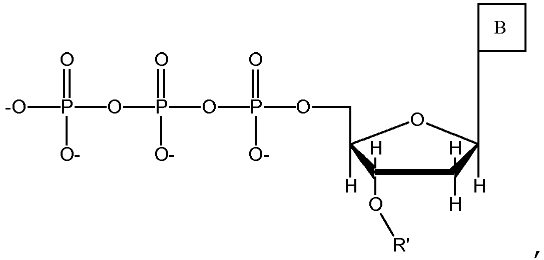 dans laquelle B est une base et est l'adénine, la guanine, la cytosine ou la thymine, et (2) R' est un hydrocarbyle, ou un hydrocarbyle substitué, ayant une masse inférieure à 300 daltons et qui peut être clivé par voie photochimique ;
(b) la détermination si l'incorporation de l'analogue de dNTP s'est produite à l'étape (a) par détection d'une augmentation de la concentration en ions hydrogène de la solution, dans laquelle une augmentation de la concentration en ions d'hydrogène indique que l'analogue de dNTP a été incorporé dans l'amorce d'ADN pour former un produit d'extension d'ADN et, si tel est le cas, la détermination à partir de l'identité de l'analogue de dNTP incorporé de l'identité du résidu nucléotidique dans l'ARN simple brin qui lui est complémentaire, déterminant ainsi l'identité du résidu nucléotidique dans l'ARN simple brin, et dans laquelle l'absence de changement dans la concentration en ions hydrogène indique que l'analogue de dNTP n'a pas été incorporé dans l'amorce d'ADN à l'étape (a) ;
(c) si aucune modification de la concentration en ions hydrogène n'a été détectée à l'étape (b), la réalisation de manière itérative des étapes (a) et (b), dans lesquelles, à chaque itération de l'étape (a), pour un résidu nucléotidique donné, dont l'identité est à déterminer, l'analogue de dNTP comprend une base qui est un type de base différent du type de base des analogues de dNTP dans chaque itération précédente de l'étape (a) pour ce résidu nucléotidique, jusqu'à ce qu'un analogue de dNTP soit incorporé dans l'amorce d'ADN pour former un produit d'extension d'ADN, et la détermination à partir de l'identité de l'analogue de dNTP incorporé de l'identité du résidu nucléotidique dans l'ARN simple brin qui lui est complémentaire, déterminant ainsi l'identité du résidu nucléotidique dans l'ARN simple brin ;
(d) si une augmentation de la concentration en ions hydrogène a été détectée et qu'un analogue de dNTP est incorporé, le traitement par la suite de l'analogue de dNTP incorporé avec de la lumière afin de cliver par voie photochimique le groupe R' de manière à remplacer son groupe R' par un atome de H, fournissant ainsi un groupe OH en 3' à l'extrémité terminale 3' du produit d'extension d' ADN ; et
(e) la réalisation de manière itérative des étapes (a) à (d), si nécessaire, pour chaque résidu nucléotidique des résidus nucléotidiques consécutifs de l'ARN simple brin à séquencer, sauf que dans chaque répétition de l'étape (a), l'analogue de dNTP est (i) incorporé dans le produit d'extension d'ADN résultant d'une itération précédente de l'étape (a) ou de l'étape (c), et (ii) complémentaire d'un résidu nucléotidique de l'ARN simple brin qui est immédiatement en 5' d'un résidu nucléotidique de l'ARN simple brin hybridé au résidu nucléotidique terminal 3' du produit d'extension d'ADN résultant d'une itération précédente de l'étape (a) ou de l'étape (c), de manière à former un produit d'extension d'ADN ultérieur, à condition que pour le dernier résidu nucléotidique à séquencer l'étape (d) soit facultative,
déterminant ainsi l'identité de chacun des résidus nucléotidiques consécutifs de l'ARN simple brin de manière à déterminer ainsi la séquence des résidus nucléotidiques consécutifs de l'ARN.

4. Procédé selon l'une quelconque des revendications 1 à 3, dans lequel une lumière UV est utilisée pour traiter le groupe R' d'un analogue de dNTP ou d'un analogue de rNTP incorporé dans une amorce ou un produit d'extension d'ADN ou d'ARN de manière à cliver par voie photochimique la fraction fixée au 3'-O de manière à remplacer le 3'-O-R' par un 3'-OH.

5. Procédé selon l'une quelconque des revendications 1 à 4, dans lequel R' est un hydrocarbyle substitué, et est un nitrobenzyle.

6. Procédé selon l'une quelconque des revendications 1 à 5, dans lequel R' est un 2-nitrobenzyle, et dans lequel une lumière UV est utilisée pour traiter le groupe R' d'un analogue de dNTP ou d'un analogue de rNTP incorporé dans une amorce ou un produit d'extension d'ADN ou d'ARN de manière à cliver par voie photochimique la fraction fixée au 3'-O de manière à remplacer le 3'-O-R' par un 3'-OH, et dans lequel de préférence la lumière est une irradiation à environ 355 nm.

7. Procédé selon l'une quelconque des revendications 1 à 6, dans lequel l'ADN ou l'ARN simple brin se trouve dans une solution dans une chambre de réaction disposée sur un capteur qui est (i) formé dans un substrat semi-conducteur et (ii) comprend un transistor à effet de champ ou un transistor à effet de champ chimique conçu pour fournir au moins un signal de sortie en réponse à une augmentation de la concentration en ions hydrogène de la solution résultant de la formation d'une liaison phosphodiester entre un dNTP ou un analogue de dNTP ou un rNTP ou un analogue de rNTP et une amorce ou un produit d'extension d'ADN ou d'ARN.

8. Procédé selon la revendication 7, dans lequel la chambre de réaction est l'une d'une pluralité de chambres de réaction disposées sur un réseau de capteurs formé dans un substrat semi-conducteur et composé d'une pluralité de capteurs, chaque chambre de réaction étant disposée sur au moins un capteur et chaque capteur du réseau comprenant un transistor à effet de champ, ou un transistor à effet de champ chimique, conçu pour fournir au moins un signal de sortie en réponse à une augmentation de la concentration en ions hydrogène de la solution résultant de la formation d'une liaison phosphodiester entre un dNTP, un analogue de dNTP, un rNTP ou un analogue de rNTP et une amorce ou un produit d'extension d'ADN ou d'ARN.

9. Procédé selon la revendication 8, dans lequel lesdits capteurs dudit réseau occupent chacun une surface de 100 µm ou moins et ont un pas de 10 µm ou moins et dans lequel chacune desdites chambres de réaction a un volume compris dans la plage de 1 µm³ à 1 500 µm³ ; dans lequel chacune desdites chambres de réaction contient au moins 10⁵ copies de l'ADN ou de l'ARN simple brin dans la solution ; ou dans lequel ladite pluralité desdites chambres de réaction et ladite pluralité desdits capteurs ont chacune un nombre supérieur à 256 000.

10. Procédé selon l'une quelconque des revendications 1 à 9, dans lequel un ou plusieurs ADN ou ARN simple brin dans la solution sont fixés à un substrat solide ; dans lequel une amorce dans la solution est fixée à un substrat solide ; dans lequel l'ADN ou l'ARN simple brin ou l'amorce est fixé(e) à un substrat solide via une chimie de cycloaddition 1,3-dipolaire azoture-alcyne ; dans lequel l'ADN ou l'ARN simple brin ou l'amorce est fixé(e) à un substrat solide via une molécule de polyéthylèneglycol ; dans lequel l'ADN ou l'ARN simple brin ou l'amorce est fixé(e) à un substrat solide via une molécule de polyéthylèneglycol et est fonctionnalisé(e) par un azoture ; dans lequel l'ADN ou l'ARN simple brin ou l'amorce est fixé(e) à un substrat solide via une liaison azido, une liaison alcynyle ou une interaction biotine-streptavidine ; dans lequel l'ADN ou l'ARN simple brin ou l'amorce est marqué(e) par un alcyne ; dans lequel l'ADN ou l'ARN simple brin ou l'amorce est fixé(e) à un substrat solide se présentant sous la forme d'une puce, d'une bille, d'un puits, d'un tube capillaire, d'une lame, d'une plaquette, d'un filtre, d'une fibre, d'un milieu poreux, d'une matrice, d'un nanotube poreux ou d'une colonne ; dans lequel l'ADN ou l'ARN simple brin ou l'amorce est fixé(e) à un substrat solide qui est un métal, de l'or, de l'argent, du quartz, de la silice, un plastique, du polypropylène, du verre, du nylon ou du diamant ; dans lequel l'ADN ou l'ARN simple brin ou l'amorce est fixé(e) à un substrat solide qui est une substance poreuse non métallique à laquelle est fixé ou imprégné un métal ou une combinaison de métaux ; dans lequel l'ADN ou l'ARN simple brin ou l'amorce est fixé(e) à un substrat solide qui est à son tour fixé à un second substrat solide ; ou dans lequel l'ADN ou l'ARN simple brin ou l'amorce est fixé(e) à un substrat solide qui est à son tour fixé à un second substrat solide qui est une puce.

11. Procédé selon l'une quelconque des revendications 1 à 10, dans lequel 1 x 10⁹ copies ou moins de l'ADN ou de l'ARN simple brin ou de l'amorce sont fixées à un substrat solide ; dans lequel 1 x 10⁸ copies ou moins de l'ADN ou de l'ARN simple brin ou de l'amorce sont fixées à un substrat solide ; dans lequel 2 x 10⁷ copies ou moins de l'ADN ou de l'ARN simple brin ou de l'amorce sont fixées à un substrat solide ; dans lequel 1 x 10⁷ copies ou moins de l'ADN ou de l'ARN simple brin ou de l'amorce sont fixées à un substrat solide ; dans lequel 1 x 10⁶ copies ou moins de l'ADN ou de l'ARN simple brin ou de l'amorce sont fixées à un substrat solide ; dans lequel 1 x 10⁴ copies ou moins de l'ADN ou de l'ARN simple brin ou de l'amorce sont fixées à un substrat solide ; ou dans lequel 1 000 copies ou moins de l'ADN ou de l'ARN simple brin ou de l'amorce sont fixées à un substrat solide.

12. Procédé selon l'une quelconque des revendications 1 à 10, dans lequel 10 000 copies ou plus de l'ADN ou de l'ARN simple brin ou de l'amorce sont fixées à un substrat solide ; dans lequel 1 x 10⁷ copies ou plus de l'ADN ou de l'ARN ou de l'amorce sont fixées à un substrat solide ; dans lequel 1 x 10⁸ copies ou plus de l'ADN ou de l'ARN simple brin ou de l'amorce sont fixées à un substrat solide ; dans lequel 1 x 10⁹ copies ou plus de l'ADN ou de l'ARN simple brin ou de l'amorce sont fixées à un substrat solide ; ou dans lequel l'ADN ou l'ARN simple brin ou l'amorce sont séparés dans des compartiments discrets, des puits discrets ou des dépressions discrètes sur une surface solide.

13. Procédé selon l'une quelconque des revendications 1 à 12, exécuté en parallèle sur une pluralité d'ADN ou d'ARN simple brin ; et dans lequel, éventuellement, les ADN ou ARN simple brin sont des matrices ayant la même séquence.

14. Procédé selon la revendication 13, comprenant en outre, après la détermination du résidu du résidu nucléotidique à l'étape (b), ou (c), selon le cas, le coiffage de manière permanente de toutes les amorces non prolongées ou de tous les produits d'extension d'ADN ou d'ARN non prolongés.

15. Procédé selon l'une quelconque des revendications 13 ou 14, dans lequel l'ADN ou l'ARN simple brin est amplifié à partir d'un échantillon d'ADN ou d'ARN avant l'étape (a) ; et dans lequel éventuellement l'ADN ou l'ARN simple brin est amplifié par une réaction en chaîne par polymérase.
